# EUROPEAN PATENT APPLICATION

(11) **EP 4 512 410 A1**
(43) Date of publication of application: **26.02.2025**
(21) Application number: 23785026.8
(22) Date of filing: 06.04.2023
(51) Int. Cl.: A61K 38/17, A61K 47/64, A61K 47/65, A61K 47/55, A61P 35/00, C07K 14/435, A61K 47/68, C12Q 1/6886, G01N 33/574, G01N 33/68

(54) **NOVEL-PEPTIDE-BASED ANTICANCER IMMUNOTHERAPEUTIC AGENT**

(30) Priority: 07.04.2022 KR 20220043200; 07.04.2022 KR 20220043573
(71) Applicant: Twinpig Biolab, Inc., Seoul 02447 (KR)
(72) Inventor: BAE, Hyunsu, Seoul 05229 (KR); HAN, Ik-Hwan, Namyangju-si Gyeonggi-do 12160 (KR); KANG, Moonkyu, Namyangju-si Gyeonggi-do 12257 (KR); LEE, Heekyung, Yongin-si Gyeonggi-do 16927 (KR); CHOI, Jeongyoon, Seoul 01775 (KR); CHOI, Ilseob, Seoul 05112 (KR); YANG, Juwon, Seoul 02451 (KR); CHOI, Hongseo, Seoul 04999 (KR); SHIN, Jin Sun, Seoul 01854 (KR)
(74) Representative: Isarpatent
(86) International application number: PCT/KR2023/004674
(87) International publication number: WO 2023/195802

(57) **Abstract**

The present disclosure relates to a novel-peptide-based anticancer immunotherapeutic agent, and to use, as an anticancer immunotherapeutic agent, of melittin, a variant thereof, analogues thereof, or a conjugate in which a drug is linked thereto. The melittin, the variant thereof or the analogues thereof, of the present disclosure, specifically bind to ITGB2, which is expressed on the cell membrane of M2 tumor-associated macrophages, so as to kill the M2 tumor-associated macrophages, and a conjugate in which a pro-apoptotic peptide or an anticancer drug is conjugated as a drug to the melittin, the variant thereof, or the analogues thereof remarkably increases apoptotic effects of the M2 tumor-associated macrophages, and thus can be used as an anticancer composition for suppressing tumor growth and metastasis.

## Description

### [Technical Field]

The present disclosure relates to a novel-peptide-based anticancer immunotherapeutic agent, and to use, as an anticancer immunotherapeutic agent, of melittin, a variant thereof, analogues thereof, or a conjugate in which a drug is linked thereto.

### [Background Art]

Conventional anti-cancer therapies have been studied to enhance the activity of immune cells in the body that directly attack or attack cancer cells. However, these anti-cancer drugs also attack other normal cells other than cancer cells, resulting in many side-effects such as hair loss, nausea, and vomiting, and cause additional reactions due to an excessive increase of immune cells. Compared to existing chemotherapy or radiation therapy, anticancer immunotherapy is a method of treating cancer by using an in vivo immune system capable of minimizing side effects. Among these anticancer immunotherapy techniques, there have been actively progressed cell therapy methods that activate therapeutic immune cells such as T cells (including CAR-T), dendritic cells, and natural killer cells outside the body and then directly inject the activated cells into the body, anticancer vaccine methods that directly activate immune cells existing in the body by injecting cancer antigens and immune-activating substances into the body, thereby increasing anticancer efficacy, etc. However, these cell therapy agents and cancer vaccines are mainly used for blood cancer-related diseases, and have a disadvantage of having very low therapeutic efficacy in most solid cancers. One of these reasons is due to microenvironmental factors that suppress the immune function around solid cancers. In fact, cells that lower the function of immune cells in the tumor microenvironment (MDSC: myeoloid-derived stromal cells, Treg: regulatory T cells, TAM: tumor-associated macrophages), immunosuppressive cytokines, and metabolites are actively working, which rapidly reduces the activity of immune-activating substances and therapeutic immune cells. Therefore, it has been important to develop a therapeutic agent having an anticancer effect by controlling only the surrounding microenvironment of the tumor cells without directly affecting tumor cells and immune cells to block nutrient supply to tumor cells and angiogenesis around the tumor cells. The tumor microenvironment is greatly considered as a therapeutic target by contributing to the proliferation and survival of malignant cells, angiogenesis, metastasis, abnormally adaptive immunity, and reduced responses to hormones and chemotherapeutic agents.

The microenvironment around the tumor consists of endothelial cells, inflammatory cells, and fibroblasts, and in the 1970s, it was found that tumor-associated macrophages (TAMs) play an important role in tumor growth. Tumor-associated macrophages play an important role in the overall tumor microenvironment, including cancer growth and metastasis, and are classified into two phenotypes of tumor-suppressive M1-type macrophages and tumor-supportive M2-type macrophages. The M1-type macrophages have a strong antigen-presenting ability, are generally activated by interferon-γ, lipopolysaccharide (LPS), and tumor necrosis factor (TNF)-α, and have proinflammatory and bactericidal effects. The M2-type macrophages are known to promote immunosuppression, tumorigenesis, and angiogenesis by releasing various extracellular matrix components, angiogenesis and chemotaxis factors. Generally, the M2-type macrophages are induced by IL-4 and IL-13, and are distinguished from M1-type tumor-associated macrophages by expressing markers such as arginase-1, mannose (MMR, CD206), scavenger receptors (SR-A, CD204), CD163, and IL-10. Tumor-associated macrophages present around tumors are closely related to the growth and metastasis of tumor cells, and it has been reported that when a large number of M2-type tumor-associated macrophages exist around tumors in cancer patients, the patient's prognosis and survival rate are poor. It has been reported that the M2-type macrophages produce cytokines such as IL-10, TGFβ, and CCL18 that promote cancer growth, and receptors such as PDL1 and B7-1/2 present on the surface of the M2-type tumor-associated macrophages suppress the antitumor activity of T cells and NK cells. Since tumor growth, differentiation, and metastasis occur actively in a microenvironment where the M2-type tumor-associated macrophages exist in large numbers, it is necessary to develop targeted therapeutic agents targeting M2-type tumor-associated macrophages.

### [Disclosure]

### [Technical Problem]

An object of the present disclosure is to provide a pharmaceutical composition for removing tumor-associated macrophages in a tumor microenvironment.

Another object of the present disclosure is to provide a pharmaceutical composition for preventing or treating cancer.

Yet another object of the present disclosure is to provide a method for treating cancer.

Yet another object of the present disclosure is to provide a use of a conjugate of the present disclosure for the preparation of a pharmaceutical composition for preventing or treating cancer.

### [Technical Solution]

In order to achieve the above aspects, the present disclosure provides a pharmaceutical composition for removing tumor-associated macrophages in a tumor microenvironment, including as an active ingredient a conjugate in which a pro-apoptotic peptide or an anticancer drug is conjugated to melittin, a variant thereof, or analogues thereof.

In addition, the present disclosure provides a pharmaceutical composition for preventing or treating cancer, including as an active ingredient a conjugate in which a pro-apoptotic peptide or an anticancer drug is conjugated to melittin, a variant thereof, or analogues thereof.

In addition, the present disclosure provides a method for treating cancer, including administering to a subject suffering from cancer a pharmaceutically effective amount of a conjugate in which a pro-apoptotic peptide or an anticancer drug is conjugated to melittin, a variant thereof, or analogues thereof.

Furthermore, the present disclosure provides a use of a conjugate in which a pro-apoptotic peptide or an anticancer drug is conjugated to melittin, a variant thereof, or analogues thereof for the preparation of a pharmaceutical composition for preventing or treating cancer.

### [Advantageous Effects]

According to the present disclosure, the melittin, the variant thereof or the analogues thereof specifically bind to ITGB2, which is expressed on the cell membrane of M2 tumor-associated macrophages, so as to kill the M2 tumor-associated macrophages, and a conjugate in which a pro-apoptotic peptide or an anticancer drug is conjugated as a drug to the melittin, the variant thereof, or the analogues thereof remarkably increases apoptotic effects of the M2 tumor-associated macrophages, and thus can be used as an anticancer composition for suppressing tumor growth and metastasis.

### [Description of Drawings]

FIG. 1A is a diagram showing the affinity of TAMpep for M2 macrophages confirmed by flow cytometry.
FIG. 1B is a graph quantifying the affinity of TAMpep for M2 macrophages (*P < 0.05 versus the M0.).
FIG. 2A is an analytical Venn diagram of M2/M0 and M1/M0 ratios, which is a diagram identifying proteins binding to TAMpep in M2 macrophages.
FIG. 2B is a diagram identifying proteins binding to TAMpep in M2 macrophages by a scatter plot analysis of a M2/M0 ratio.
FIG. 2C is a diagram identifying proteins binding to TAMpep in M2 macrophages by a scatter plot analysis of a M1/M0 ratio.
FIG. 2D is a diagram identifying proteins binding to TAMpep in M2 macrophages by a scatter plot analysis of a M2/M1 ratio.

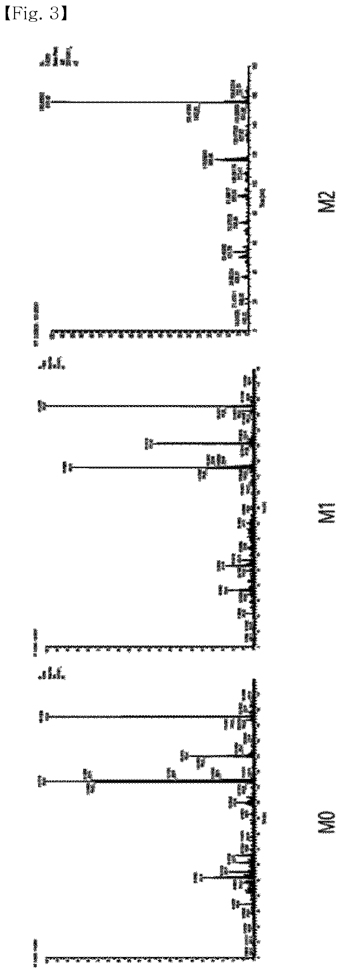

FIG. 3 is a diagram illustrating LC-MS chromatogram data obtained by reacting TAMpep826-biotin with cell membrane proteins extracted from M0, M1, and M2 macrophages, respectively.
FIG. 4 is a diagram showing identification of target proteins of TAMpep826 using LC-MS/MS proteomics.
FIG. 5 is a diagram showing quantitative RT-PCR conditions.
FIG. 6 is a diagram confirming ITGB2 mRNA expression in M2 macrophages.
FIG. 7 is a diagram confirming ITGB2 protein expression in M2 macrophages.
FIG. 8 is a diagram confirming ITGB2 protein expression in M2 macrophages by immunofluorescence analysis.
FIG. 9 is a diagram confirming ITGB2 expression on the cell membrane of M2 macrophages.
FIG. 10 is a diagram confirming interaction between TAMpep826 and ITGB2 in M2 macrophages by pull-down assay.
FIG. 11 is a diagram confirming interaction between TAMpep826 and ITGB2 in M2 macrophages by immunofluorescence analysis.
FIGS. 12 and 13 are diagrams confirming binding sites of TB511 and ITGB2 through docking simulation.
FIG. 14 is a schematic diagram showing that an anti-CD18 antibody used in the fabrication of an ITGB2 binding ADC of the present disclosure specifically binds to an active conformation (extended-open form) of CD18 in M2 TAM.
FIG. 15 is a diagram showing ITGB2 immobilized on the surface of a sensor chip.
FIG. 16 is a diagram confirming binding affinity between anti-ITGB2 antibody and ITGB2 protein by SPR.
FIG. 17 is a diagram confirming binding affinity between Melittin-dKLA and ITGB2 protein by SPR.
FIG. 18 is a diagram confirming the mRNA expression of ITGB2 in an ITGB2 expression suppression cell model established through Crispr/cas9 in M2 macrophages.
FIG. 19 is a diagram confirming an apoptosis reduction effect of Melittin-dKLA by suppressing ITGB2 expression in M2 macrophages.
FIG. 20 is a diagram confirming an apoptosis-induction reduction effect of TB511 (TAMpep826-dKLA) by suppressing ITGB2 expression in M2 macrophages.
FIG. 21 is a diagram confirming an apoptosis-induction reduction effect of TB511 by an ITGB2 antibody in M2 macrophages.
FIGS. 22 and 23 are diagrams confirming effects of TB511 in breast cancer 3D organoids.
FIGS. 24 and 25 are diagrams confirming effects of TB511 in lung cancer 3D organoids.
FIG. 26 is a diagram confirming an effect of M-DM1 in lung cancer 3D organoids.
FIGS. 27 and 28 are diagrams confirming effects of TB511 in anticancer drug-resistant lung cancer 3D organoids.
FIGS. 29 and 30 are diagrams confirming an effect of TB511 in anticancer drug-resistant prostate cancer 3D organoids.
FIG. 31 is a diagram confirming an effect of CD18-DM1 in breast cancer 3D organoids.
FIG. 32 is a diagram confirming an effect of CD18-MMAE in breast cancer 3D organoids.
FIGS. 33 and 34 are diagrams confirming effects of ADCs (CD18-DM1 and CD18-MMAE) in lung cancer 3D organoids.
FIGS. 35 and 36 are diagrams confirming effects of ADCs (CD18-DM1 and CD18-MMAE) in anticancer drug-resistant lung cancer 3D organoids.
FIGS. 37 and 38 are diagrams confirming effects of ADCs (CD18-DM1 and CD18-MMAE) in anticancer drug-resistant prostate cancer 3D organoids.

### [Best Mode of the Invention]

Hereinafter, embodiments of the present disclosure will be described in detail with reference to the accompanying drawings. However, the following embodiments are presented as examples for the present disclosure, and when it is determined that a detailed description of well-known technologies or configurations known to those skilled in the art may unnecessarily obscure the gist of the present disclosure, the detailed description thereof may be omitted, and the present disclosure is not limited thereto. Various modifications and applications of the present disclosure are possible within the description of claims to be described below and the equivalent scope interpreted therefrom.

Terminologies used herein are terminologies used to properly express preferred embodiments of the present disclosure, which may vary according to a user, an operator's intention, or customs in the art to which the present disclosure pertains. Therefore, these terminologies used herein will be defined based on the contents throughout the specification. Throughout the specification, unless explicitly described to the contrary, when a certain part "comprises" a certain component, it will be understood to further include another component but not the exclusion of other components.

All technical terms used in the present disclosure, unless otherwise defined, are used as the meaning as commonly understood by those skilled in the related art of the present disclosure. In addition, although preferred methods and samples are described herein, similar or equivalent methods and samples thereto are also included in the scope of the present disclosure. The contents of all publications disclosed as references in this specification are incorporated in the present disclosure.

Throughout the present specification, general one-letter or three-letter codes for naturally existing amino acids are used, and generally allowed three-letter codes for other amino acids, such as α-aminoisobutyric acid (Aib) and N-methylglycine (Sar) are also used. The amino acids mentioned herein as abbreviations are also described as follows according to the IUPAC-IUB nomenclature.

Alanine: A; Arginine: R; Asparagine: N; Aspartic acid: D; Cysteine: C; Glutamic acid: E; Glutamine: Q; Glycine: G; Histidine: H; Isoleucine: I; Leucine: L; Lysine: K; Methionine: M; Phenylalanine: F; Proline: P; Serine: S; Threonine: T; Tryptophan: W; Tyrosine: Y; and Valine: V.

In one aspect, the present disclosure relates to a pharmaceutical composition for removing tumor-associated macrophages (TAMs) in a tumor microenvironment, including as an active ingredient a conjugate in which a pro-apoptotic peptide or an anticancer drug is conjugated to melittin, a variant thereof, or analogues thereof.

In one embodiment, the tumor-associated macrophages may be M2 tumor-associated macrophages expressing Integrin beta 2 (IGBT2).

In one embodiment, the composition may remove only M2 macrophages without removing M1 macrophages.

In one embodiment, the melittin may include an amino acid sequence of SEQ ID NO: 1.

In one embodiment, the variant of melittin may be a peptide in which amino acids at positions 1 to 7 are deleted from the amino acid sequence of SEQ ID NO: 1, and may include an amino acid sequence of SEQ ID NO: 2.

In one embodiment, the ITGB2 may include an amino acid sequence of SEQ ID NO: 7 or 8.

In one embodiment, the melittin, the variant thereof or the analogues thereof may specifically bind to an active conformation in which ITGB2 (CD18) is extended.

In one embodiment, the melittin, the variant thereof or the analogues thereof may bind to amino acids at positions 466 to 565 of ITGB2.

In one embodiment, the composition may include a peptide including an amino acid sequence of SEQ ID NO: 5 or 6.

In one embodiment, the conjugate in which the pro-apoptotic peptide or the anticancer drug is conjugated to the melittin, the variant thereof or the analogues thereof may be a peptide-drug conjugate (PDC). The PDC may be Melittin-dKLA including an amino acid sequence of SEQ ID NO: 5, may be TB511 including an amino acid sequence of SEQ ID NO: 6, or may be M-DM1 in which a drug DM1 is conjugated to maleimide-modified melittin.

In one embodiment, LEU(6) of TB511 may bind to LEU(528), THR(538), LEU(556), CYS(557) and PHE(558) sites of ITGB2, and TRP(12) of TB511 may bind to GLU(466), ILE(469), CYS(470) and ARG(471) of ITGB2.

In one embodiment, the pro-apoptotic peptide or anticancer drug may bind to the melittin, the variant thereof or the analogues thereof covalently via a linker or non-covalently without a linker.

In one embodiment, the linker may include an amino acid sequence of SEQ ID NO: 3.

In one embodiment, the melittin, the variant thereof or the analogues thereof may further include a targeting sequence, a tag, a labeled residue or an amino acid sequence designed for a specific purpose to increase the half-life or stability.

In one embodiment, the pro-apoptotic peptide may be selected from the group consisting of KLA, alpha-defensin-1, BMAP-28, Brevenin-2R, Buforin IIb, cecropin A-Magainin 2 (CA-MA-2), Cecropin A, Cecropin B, chrysophsin-1, D-K6L9, Gomesin, Lactoferricin B, LLL27, LTX-315, Magainin 2, Magainin II-bombesin conjugate (MG2B), Pardaxin, and combinations thereof, more preferably D-type isomer KLA, and may include an amino acid sequence of SEQ ID NO: 4.

In one embodiment, the anticancer drug may be selected from the group consisting of 7-ethyl-10-hydroxy-camptothecin (SN-38), daunorubicin, doxorubicin, epirubicin, idarubicin, pixantrone, sabarubicin, valrubicin, paclitaxel, docetaxel, mechloethamine, chlorambucil, phenylalanine, mustard, cyclophosphamide, ifosfamide, carmustine (BCNU), lomustine (CCNU), Streptozotocin, busulfan, thiotepa, cisplatin, carboplatin, dactinomycin (actinomycin D), plicamycin, mitomycin C, vincristine, vinblastine, teniposide, topotecan, iridotecan, uramustine, melphalan, bendamustine, dacarbazine, temozolomide, altretamine, duocarmycin, nedaplatin, oxaliplatin, satraplatin, triplatin tetranitrate, 5-fluorouracil, 6-mercaptopurine, capecitabine, cladribine, clofarabine, cystarbine, floxuridine, fludarabine, gemcitabine, hydroxyurea, methotrexate, pemetrexed, pentostatin, thioguanine, etoposide, mitoxantrone, izabepilone, vindesine, vinorelbine, estramustine, maytansine, mertansine (DM1), DM4, dolastatin, auristatin E, auristatin F, monomethyl auristatin E (MMAE), monomethyl auristatin F, and derivatives thereof, and may be DM1 or MMAE.

In one embodiment, the pro-apoptotic peptide or the anticancer drug may be linked to the melittin, the variant thereof or the analogues thereof by a chemical linker or a direct bond, and the chemical linker may be linked to the pro-apoptotic peptide or the anticancer drug through an amine group, a carboxyl group or a sulfhydryl group on the melittin, the variant thereof or the analogues thereof.

In one embodiment, the chemical linker may include a functional group selected from carbodiimide, N-hydroxysuccinimide ester (NHS ester), imidoester, pentafluorophenyl ester, hydroxymethyl phosphine, maleimide, haloacetyl, pyridyldisulfide, thiosulfonate, vinylsulfone, and combinations thereof at both terminals, and may be selected from 1-ethyl-3-(3-dimethylaminopropyl) carbodiimide (EDC), N,N'-dicyclohexylcarbodiimide (DCC), succinimidyl acetylthioacetate (SATA), Sulfo-SMCC (sulfosuccinimidyl-4-(NDmaleimidomethyl)cyclohexane-1-carboxylate), DMA (dimethyl adipimidate·2HCl), DMP (dimethylpimelimidate·2HCl), DMS (dimethyl Suberimidate·2HCl), DTBP (dimethyl 3,3'-dithiobispropionimidate·2HCl), sulfo-SIAB (sulfosuccinimidyl (4-iodoacetyl)aminobenzoate), SIAB(succinimidyl (4-iodoacetyl)aminobenzoate), SBAP (succinimidyl 3-(bromoacetamido)propionate), SIA(succinimidyl iodoacetate), SM(PEG)n (succinimidyl-([N-maleimidopropionamido]-#ethyleneglycol ester, n = 2, 4, 6, 8, 12, or 24), SMCC(succinimidyl-4-(N-Dmaleimidomethyl)cyclohexane-1-carboxylate), LCSMCC (succinimidyl 4-(N-maleimidomethyl)cyclohexane-1-carboxy-(6-amidocaproate)), sulfo-EMCS (N-εester), EMCS(N-εsulfo-GMBS(N-γester), GMBS(N-γ ester), sulfo-KMUS (N-κester), sulfo-MBS (m-maleimidobenzoyl-Nhydroxysulfosuccinimide ester), MBS (m-maleimidobenzoyl-N-hydroxysuccinimide ester), sulfo-SMPB (sulfosuccinimidyl 4-(p-maleimidophenyl)butyrate), SMPB (succinimidyl 4-(pmaleimidophenyl)butyrate), AMAS (N-α-imidoacet-oxysuccinimide ester), BMPS(N-β-maleimidopropyloxysuccinimide ester), SMPH(succinimidyl 6-[(β-maleimidopropionamido)hexanoate]), PEG12-SPDP(2-pyridyldithioltetraoxaoctatriacontane-N-hydroxysuccinimide), PEG4-SPDP, sulfo-LCSPDP(sulfosuccinimidyl 6-[3'-(2-pyr) idyldithio)propionamido]hexanoate), SPDP(succinimidyl 3-(2-pyridyldithio)propionate), LC-SPDP(succinimidyl 6-[3'-(2-pyridyldithio)propionamido]hexanoate), SMPT(4-succinimidyloxycarbonyl-alphamethyl-alpha(2-pyridyldithio)toluene), DSS(disuccini) midylsuberate), BS(PEG)5(bis(succinimidyl) penta(ethylene glycol)), BS(PEG)9(bis(succinimidyl)nona(ethylene glycol)), BS3(bis[sulfosuccinimidyl] suberate), BSOCOES(bis[2-(succinimidooxycarbonyloxy)ethyl]sulfone), PDPH(3-(2-pyridyldithio)propionyl hydrazide), DSG (disuccinimidyl glutarate), DSP (dithiobis[succinimidyl propionate]), BM(PEG)n(1,8-bismaleimido-ethyleneglycol, n=2 or 3), BMB(1,4-bismaleimidobutane), BMDB(1,4-bismaleimidyl-2,3-dihydroxybutane), BMH(bismaleimidohexane), BMOE (bismaleimidoethane), DTME (dithiobismaleimidoethane), TMEA (tris(2-maleimidoethyl)amine), DSS (disuccinimidyl suberate), DST (disuccinimidyl tartarate), DTSSP (3,3'-dithiobis[sulfosuccinimidylpropionate]), EGS (ethylene glycol bis[succinimidylsuccinate]), sulfo-EGS (ethylene glycol bis[sulfosuccinimidylsuccinate]) and TSAT (tri s-succinimidylaminotriacetate), DFDNB (1,5-difluoro-2,4-dinitrobenzene), and combinations thereof.

In the present disclosure, it is preferable that the peptide has the amino acid sequence, but is not limited thereto. According to a preferred embodiment of the present disclosure, the peptide preferably has a high ratio of the amino acid of 50% or more, preferably 60% or more, more preferably 70% or more, more preferably 80% or more, more preferably 90% or more, and most preferably 100%.

In the present disclosure, the peptide of the present disclosure may further include an amino acid sequence designed for a specific purpose of increasing a targeting sequence, a tag, labeled residues, a half-life or peptide stability. In addition, the peptide of the present disclosure may be linked to coupling partners such as effectors, drugs, prodrugs, toxins, peptides, and delivery molecules.

In the present disclosure, the peptide may be prepared in the form of a pharmaceutically acceptable salt. Specifically, the salt may be formed by adding acids, for example, an inorganic acid (e.g., hydrochloric acid, hydrobromic acid, phosphoric acid, nitric acid, sulfuric acid, etc.), an organic carboxylic acid (e.g., acetic acid, haloacetic acids such as trifluoroacetic acid, propionic acid, maleic acid, succinic acid, malic acid, citric acid, tartaric acid, salicylic acid), and an organic sulfonic acid including an acidic sugar (glucuronic acid, galacturonic acid, gluconic acid, ascorbic acid), an acidic polysaccharide (e.g., hyaluronic acid, chondroitin sulfate, arginic acid), a sulfonic acid sugar ester such as chondroitin sulfate (e.g., methanesulfonic acid, p-toluene sulfonic acid).

The term "conjugate" of the present disclosure refers to a conjugate in which the pro-apoptotic peptide or the anticancer drug is conjugated to the melittin, the variant thereof, or the analogue peptide thereof, which may target M2-type tumor-associated macrophages. The conjugate may bind to targeting M2-type macrophages to damage the mitochondria of macrophages and thus suppress tumor growth and metastasis and selectively suppress angiogenesis in the surrounding area to inhibit cancer.

That is, the conjugate of the present disclosure may have improved anticancer activity compared to an anticancer drug, but is not limited thereto.

In the present disclosure, the conjugate may link the pro-apoptotic peptide to the melittin, the variant thereof, or the analogues thereof via a GGGGS linker (SEQ ID NO: 3), or may link the anticancer drug such as Doxorubicin, Methotrexate, Entinostat, Cladribine, Pralatrexate, and Lorlatinib via a SPDP linker. In addition, among the anticancer drugs, Maytansine DM1, Maytansine DM3, and Maytansine DM4 may be directly linked and conjugated without a linker, but are not limited thereto.

That is, the conjugate of the present disclosure may be in a form in which the melittin, the variant thereof, or the analogues thereof is conjugated to an anticancer drug via a chemical linker or directly, but is not limited thereto.

The term "melittin (MEL)" of the present disclosure is a peptide that constitutes the main component of bee venom. The term "bee venom (BV)" used herein is a mixture of acidic and basic secretions fabricated in the abdomen of Apis mellifera and has a colorless bitter liquid form, and main ingredients thereof include melittin, apamin, and mast cell degranulating (MCD) peptide as peptides, phospholipase A2 (PLA2) as an enzyme, and the like, and further include various trace ingredients. Therefore, the melittin of the present disclosure may be isolated from the bee venom of Apis mellifera, but is not limited thereto.

The term "peptide" used herein refers to an amino acid polymer, which may include not only natural amino acids but also non-protein amino acids as components.

As used herein, the term "variant" refers to a corresponding amino acid sequence that contains at least one amino acid difference (substitution, insertion or deletion) compared to a reference substance. In specific embodiments, the "variant" has high amino acid sequence homology and/or conservative amino acid substitution, deletion and/or insertion compared to a reference sequence.

As used herein, the term "peptide analogues" may include analogues in which the side chain or the alpha-amino acid backbone of an amino acid is substituted with one or more other functional groups. Examples of side chain or backbone modified peptide analogues may include hydroxyproline or N-methyl glycine "peptoids" in which a pyrrolidine ring is substituted with a hydroxy group, but are not limited thereto. Types of peptide analogues are well known in the art.

The protein variant according to the present disclosure is interpreted to include a variant in which an amino acid residue is conservatively substituted at a specific amino acid residue position.

As used herein, the "conservative substitution" means a modification of a variant that includes substituting one or more amino acids with amino acids having similar biochemical properties that do not cause loss of biological or biochemical functions of the melittin protein variant. The "conservative amino acid substitution" is a substitution that replaces an amino acid residue with an amino acid residue having a similar side chain. Kinds of amino acid residues having similar side chains are defined in the art and well known. These kinds include amino acids having basic side chains (e.g., lysine, arginine, histidine), amino acids having acidic side chains (e.g., aspartic acid, glutamic acid), amino acids having uncharged polar side chains (e.g., glycine, asparagine, glutamine, serine, threonine, tyrosine, cysteine), amino acids having non-polar side chains (e.g., alanine, valine, leucine, isoleucine, proline, phenylalanine, methionine, tryptophan), amino acids having beta-branched side chains (e.g., threonine, valine, isoleucine), and amino acids having aromatic side chains (e.g., tyrosine, phenylalanine, tryptophan, histidine).

Specifically, the peptides according to the present disclosure may be prepared by a standard synthetic method, a recombinant expression system, or any other methods in the art. Accordingly, the peptides according to the present disclosure may be synthesized by many methods including, for example, the following methods:
(a) a method of synthesizing a peptide by stages or by fragment assembly by means of a solid phase or liquid phase method and isolating and purifying a final peptide product;
(b) a method of expressing a nucleic acid construct encoding the peptide in a host cell, and recovering an expression product from a host cell culture; or
(c) a method of performing the expression of the nucleic acid construct encoding the peptide within a cell-free tube, and recovering an expression product; or
a method of obtaining fragments of the peptide in any combination of (a), (b) and (c), and then linking the fragments to obtain a peptide, and recovering the corresponding peptide.

In one aspect, the present disclosure provides a pharmaceutical composition for preventing or treating cancer, including as an active ingredient a conjugate in which a pro-apoptotic peptide or an anticancer drug is conjugated to melittin, a variant thereof, or analogues thereof.

In one embodiment, the cancer may be any one or more selected from the group consisting of brain tumor, melanoma, myeloma, non-small cell lung cancer, oral cancer, liver cancer, stomach cancer, colon cancer, breast cancer, TNBC (Triple Negative Cancer), lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph adenocarcinoma, bladder cancer, gallbladder cancer, endocrine adenocarcinoma, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem glioma and pituitary adenoma, and may be anticancer drug-resistant cancer.

In one embodiment, the composition may be administered to a patient in whom the expression of ITGB2 is upregulated in M2 macrophages, compared to M0 macrophages and M1 macrophages.

In one embodiment, the composition may be a targeted anticancer drug.

The pharmaceutical composition of the present disclosure may be used as a single therapy, but may also be used in combination with other conventional biological therapy, chemotherapy or radiation therapy, and may treat more effectively cancer in the case of such concurrent therapy. In the case of using the present disclosure for preventing or treating cancer, a chemotherapeutic agent that may be used with the composition includes cisplatin, carboplatin, procarbazine, mechlorethamine, cyclophosphamide, ifosfamide, melphalan, chlorambucil, bisulfan, nitrosourea, dactinomycin, daunorubicin, doxorubicin, bleomycin, plicomycin, mitomycin, etoposide, tamoxifen, taxol, transplatinum, 5-fluorouracil, vincristin, vinblastin, methotrexate, and the like. Radiation therapy that may be used together with the composition of the present disclosure includes X-ray irradiation and γ-ray irradiation.

As used herein, the term "prevention" means all actions that inhibit or delay the occurrence, spread, and recurrence of cancer by administration of the pharmaceutical composition according to the present disclosure.

The therapeutically effective amount of the composition of the present disclosure may vary depending on many factors, for example, an administration method, a target site, a condition of a patient, and the like. Accordingly, when used in the human body, a dose should be determined as an appropriate amount in consideration of both safety and efficiency. It is also possible to estimate the amount used in humans from the effective amount determined through animal experiments. These matters to be considered when determining the effective amount are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present disclosure is administered in a pharmaceutically effective amount. As used herein, the "pharmaceutically effective amount" refers to an amount enough to treat the disease at a reasonable benefit/risk ratio applicable to medical treatment and enough not to cause side effects. An effective amount level may be determined according to factors including patient's health status, the type and severity of a disease of a patient, the activity of a drug, the sensitivity to a drug, an administration method, a time of administration, a route of administration, an emission rate, duration of treatment, and simultaneously used drugs, and other factors well-known in the medical field. The composition of the present disclosure may be administered as an individual therapeutic agent or in combination with other therapeutic agents, and may be administered sequentially or simultaneously with conventional therapeutic agents, and may be administered singly or multiply. It is important to administer an amount capable of obtaining a maximum effect with a minimal amount without side effects by considering all the factors, which may be easily determined by those skilled in the art.

The pharmaceutical composition of the present disclosure may include a carrier, a diluent, an excipient, or a combination of two or more thereof, which are commonly used in biological agents. As used herein, the term "pharmaceutically acceptable" refers to exhibiting non-toxic properties to normal cells or humans exposed to the composition. The carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these components, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using a suitable method in the art or a method disclosed in Remington's Pharmaceutical Science (Mack Publishing Company, Easton PA, 18th, 1990).

In one embodiment, the pharmaceutical composition may be one or more formulations selected from the group including oral formulations, external formulations, suppositories, sterile injection solutions and sprays.

The composition of the present disclosure may include a carrier, a diluent, an excipient, or a combination of two or more thereof, which are commonly used in biological agents. The pharmaceutically acceptable carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these ingredients, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using a suitable method in the art or a method disclosed in Remington's Pharmaceutical Science (Mack Publishing Company, Easton PA, 18th, 1990).

The composition of the present disclosure may further contain one or more active ingredients exhibiting the same or similar functions. The composition of the present disclosure includes 0.0001 to 10 wt%, preferably 0.001 to 1 wt% of the protein, based on the total weight of the composition.

The pharmaceutical composition of the present disclosure may further include a pharmaceutically acceptable additive. At this time, the pharmaceutically acceptable additive may be used with starch, gelatinized starch, microcrystalline cellulose, lactose, povidone, colloidal silicon dioxide, calcium hydrogen phosphate, lactose, mannitol, syrup, gum arabic, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, sucrose, dextrose, sorbitol, talc and the like. The pharmaceutically acceptable additive according to the present disclosure is preferably included in an amount of 0.1 parts by weight to 90 parts by weight based on the composition, but is not limited thereto.

The composition of the present disclosure may be administered parenterally (e.g., applied intravenously, subcutaneously, intraperitoneally or topically) or orally according to a desired method, and the dose may vary depending on the weight, age, sex, and health condition of a patient, a diet, an administration time, an administration method, an excretion rate, the severity of a disease, etc. A daily dose of the composition according to the present disclosure is 0.0001 to 10 mg/ml, preferably 0.0001 to 5 mg/ml, and more preferably administered once to several times a day.

Liquid formulations for oral administration of the composition of the present disclosure correspond to suspensions, internal solutions, emulsions, syrups, etc., and may include various excipients, such as wetting agents, sweeteners, fragrances, preservatives, and the like, in addition to water and liquid paraffin, which are commonly used simple diluents. Formulations for parenteral administration include sterilized aqueous solutions, non-aqueous solvents, suspensions, emulsions, lyophilized agents, suppositories, and the like.

In one aspect, the present disclosure relates to a method for removing M2-type tumor-associated macrophages, including treating M2 tumor-associated macrophages in a test tube with the composition of the present disclosure.

In one aspect, the present disclosure relates to a composition for diagnosing cancer including the composition of the present disclosure.

In one aspect, the present disclosure relates to a kit for diagnosing cancer including a composition for diagnosing cancer of the present disclosure.

In the present disclosure, the term 'kit for diagnosing cancer' refers to a kit including the composition for diagnosing cancer of the present disclosure. Accordingly, the expression 'kit for diagnosing cancer' can be used interchangeably or in combination with the 'composition for diagnosing cancer'. As used herein, the term 'diagnosis' refers to determining the susceptibility of a subject to a specific disease or disorder, determining whether a subject currently has a specific disease or disorder, determining the prognosis of a subject suffering from a specific disease or disorder (e.g., identifying a pre-metastatic or metastatic cancer state, determining a stage of cancer, or determining the responsiveness of cancer to treatment), or therametrics (e.g., monitoring the condition of a subject to provide information about treatment efficacy).

The melittin, the variant thereof or the analogues thereof of the present disclosure specifically bind to ITGB2 expressed on the cell membrane of M2 tumor-associated macrophages to kill M2 tumor-associated macrophages by the melittin, the variant thereof or the analogues thereof or a drug (pro-apoptotic peptide or anticancer drug) linked thereto, thereby suppressing the growth and metastasis of tumors and selectively suppressing angiogenesis in the vicinity, thereby exhibiting anticancer activity, and thus can be used as theranostic agents for the diagnosis of cancer that have anticancer activity and specifically bind to M2 tumor-associated macrophages. In addition, it can be used as an anticancer adjuvant administered simultaneously, separately, or sequentially with anticancer drugs. Furthermore, since the melittin, the variant thereof or the analogues thereof specifically bind to M2 tumor-associated macrophages and thus can also be used as drug delivery vehicles targeting the melittin, the variant thereof or the analogues thereof.

In one aspect, the present disclosure relates to a method for providing information for diagnosing cancer, including contacting a biological sample isolated from a subject with a composition of the present disclosure; and comparing the biological sample with a normal control sample.

In one embodiment, the method may further include determining that the test subject is cancer if the level of M2 tumor-associated macrophages in the biological sample isolated from the subject is higher than that in the normal control sample.

As used herein, the term "sample" refers to a biological sample obtained from a subject or patient. Sources of the biological sample may be fresh, frozen and/or preserved organ or tissue samples or solid tissue from biopsies or aspirates; blood or any blood component; and cells at any time point of conception or development in a subject.

In one aspect, the present disclosure relates to a method for treating cancer, including administering to a subject suffering from cancer a pharmaceutically effective amount of a conjugate in which a pro-apoptotic peptide or an anticancer drug is conjugated to melittin, a variant thereof, or analogues thereof.

In one embodiment, the subject may be a patient in whom the expression of ITGB2 is upregulated in M2 macrophages, compared to M0 macrophages and M1 macrophages.

In one aspect, the present disclosure relates to a use of a conjugate in which a pro-apoptotic peptide or an anticancer drug is conjugated to melittin, a variant thereof, or analogues thereof for the preparation of a pharmaceutical composition for preventing or treating cancer.

Hereinafter, the present disclosure will be described in more detail with reference to the following Examples. However, the following Examples are only intended to embody the contents of the present disclosure, and the present disclosure is not limited thereto.

### [Modes of the Invention]

### Example 1. Synthesis of peptides targeting M2 tumor-associated macrophages (M2 TAMs)

Melittin and TAMpep826 in Table 1 below were synthesized and supplied by GenScript (Piscataway, NJ, USA), and dissolved in D-PBS at 5 mg/ml for use in subsequent experiments.

**[Table 1]**

| Name | Sequence | SEQ ID NO: | Description |
|---|---|---|---|
| Melittin | GIGAVLKVLTTGLP ALISWIKRKRQQ | 1 | Melittin peptide |
| TAMpep8 26 | VLTTGLPALISWIKR KRQQ | 2 | Peptide in which first 7 amino acids of melittin are removed |

### Example 2. M2 TAM targeting analysis

### 2-1. Confirmation of affinity of melittin to M2 TAM

To confirm whether the melittin fabricated in Example 1 specifically bound to M2 tumor-associated macrophages (M2 TAMs), a human monocyte cell line THP-1 was differentiated into M0, M1, and M2 macrophages, and treated with FITC-conjugated melittin, and then confirmed by flow cytometry. Specifically, THP-1 cells were treated with 100 nM phorbol 12-myristate 13-acetate (PMA) at 37°C for 24 hours (M0 macrophages), and the differentiated M0 macrophages were incubated with 100 nM LPS and 20 ng/ml IFN-γ at 37°C for 72 hours to be differentiated into M1 macrophages (M1), and incubated with 20 ng/ml IL-4 and IL-13 at 37°C for 72 hours to be differentiated into M2 macrophages (M2). The differentiated cells were treated with 50 nM FITC-conjugated melittin for 1 hour, detected with BD FACS CantoII instruments (BD biosciences, San Jose, CA, USA), and analyzed with FlowJo software (BD biosciences).

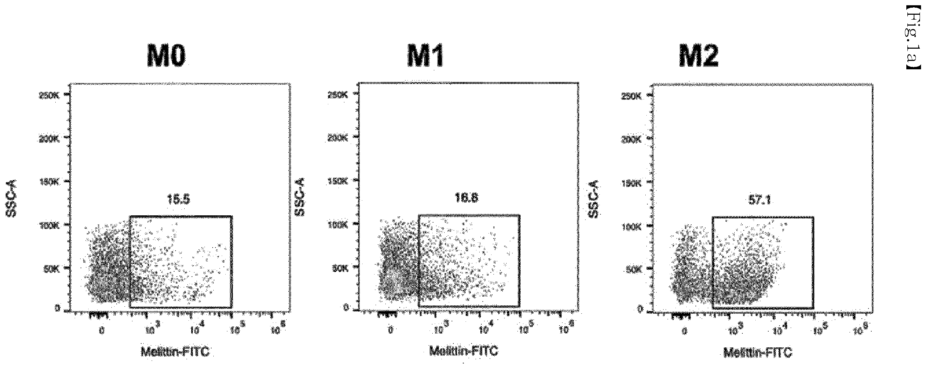

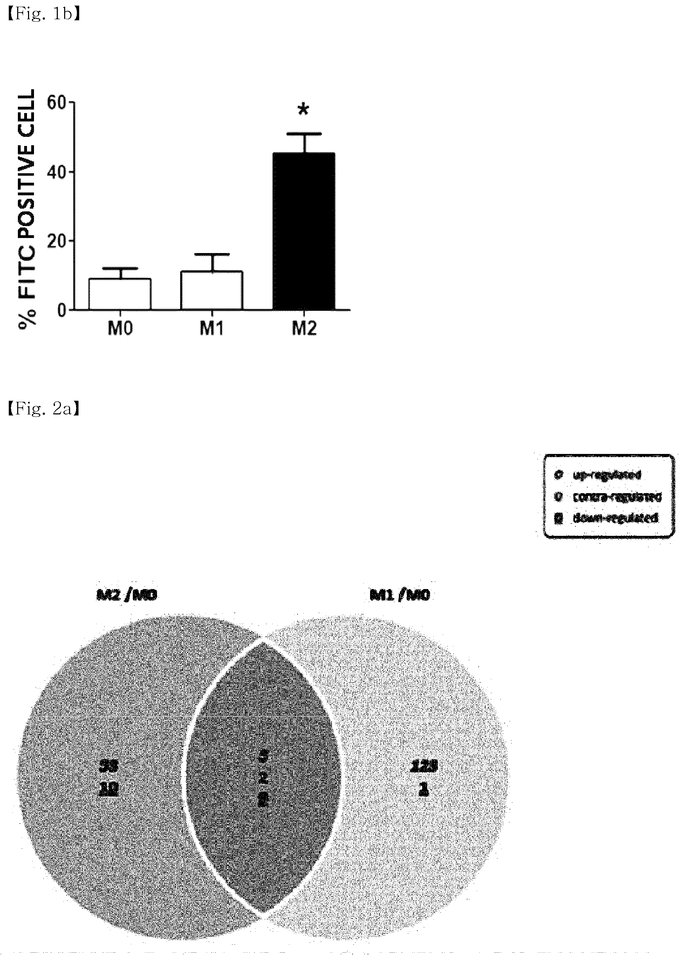

As a result, FITC-positive cells were significantly increased in M2 macrophages compared to M0 and M2 macrophages (FIGS. 1A and 1B). Through this, it was confirmed that melittin preferentially bound to M2 macrophages.

### 2-2. Identification of M2 TAM binding target protein of melittin

To determine which protein of M2 macrophages was involved in the specific binding of melittin to M2 macrophages, biotin-conjugated melittin (melittin-biotin) was synthesized. THP-1 cells (5 × 10⁶ cells) were differentiated into M0, M1, and M2 macrophages, and then collected using a scraper, washed by centrifugation (300 × g, 5 min), and cell membrane proteins of macrophages were obtained using a cell membrane protein extraction kit (Thermo Fisher scientific). The proteins reacted with melittin-biotin (200 µg) in which biotin bound to melittin at room temperature (20 to 24°C) for 1 hour, and then the proteins bound to melittin were obtained using a streptavidin resin (Thermo Fisher scientific). To remove the salt, 100 µl each of 100% methanol, 0.1% formic acid, and 80% CAN were added to an 18 Micro Spin-Column, and the sample was loaded onto the prepared column, and added with 50 µl of 0.1% formic acid to remove unnecessary substances. Thereafter, 100 µl of 80% CAN was added to elute the peptides. After desalting, the solution was completely removed by drying with a Speed-vac, and analyzed by LC-MS/MS using a UPLC-Exactive equipment (Thermo Fisher Scientific) under the conditions shown in Table 2 below. Data were analyzed for proteins upregulated in M1 and M2 macrophages compared to M0 macrophages using MaxQuant and Mass Profiler Professional (MPP). For data analysis, LC-MS/MS data for each sample were analyzed using Proteome Discoverer, and performed by LFQ (Label-Free Quantification) using a human database of Uniprot as the database. Modifications included Oxidation (M), Carbamyl (N-term), and Carbamidomethyl (C), and the Baseline option was set to none.

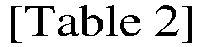

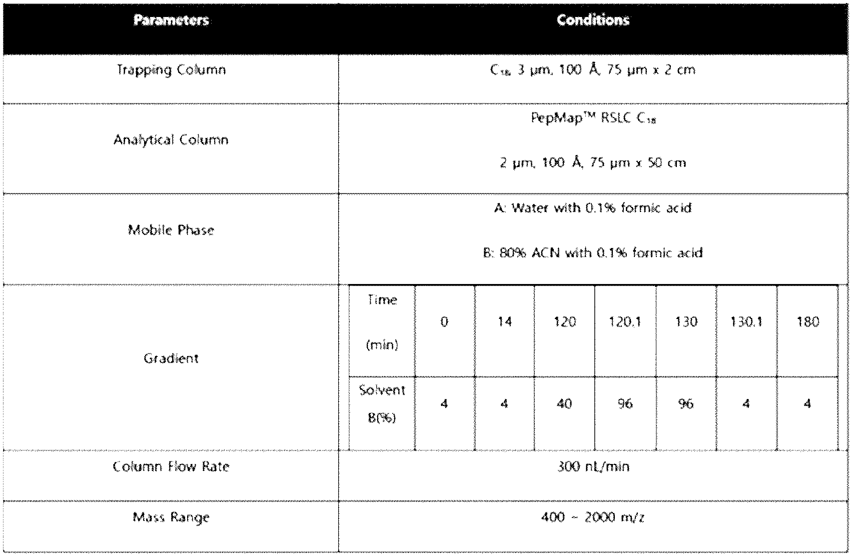

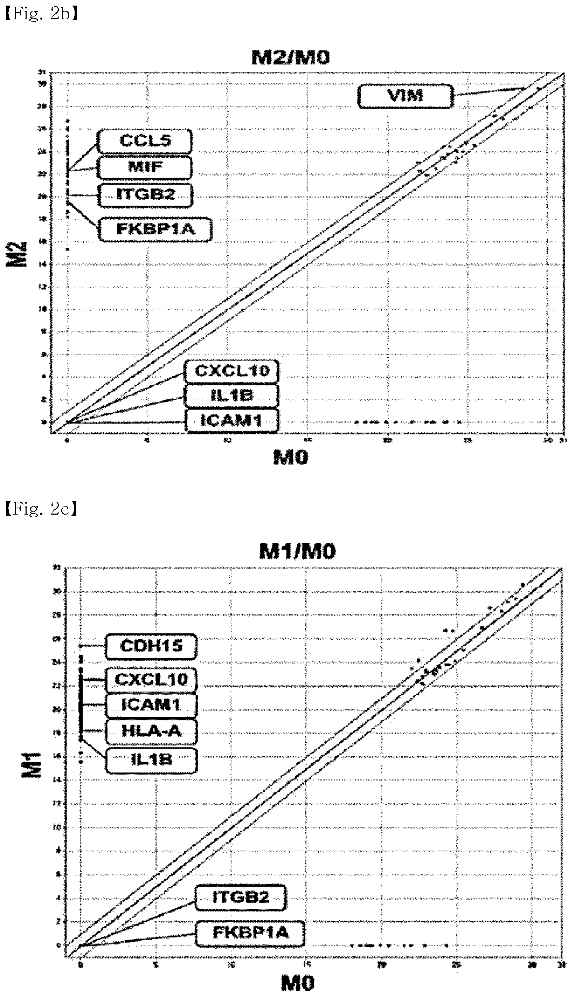

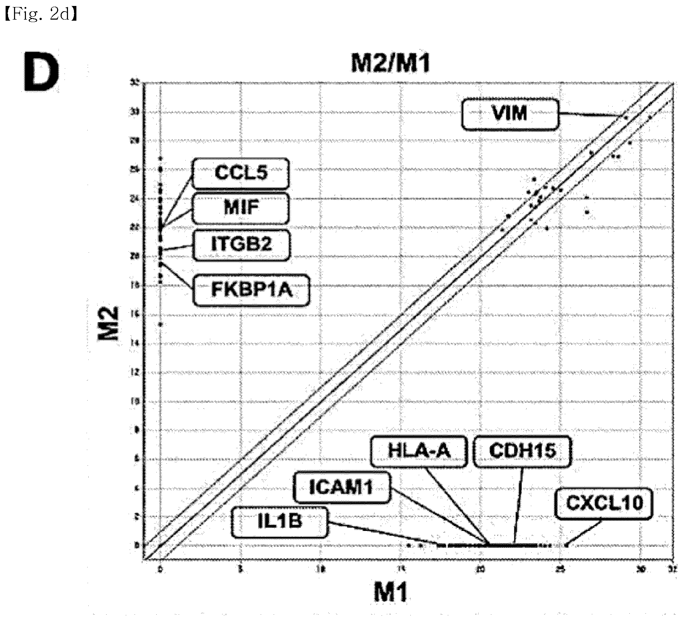

As a result, 53 proteins were upregulated in M2/M0 and 123 proteins were upregulated in M1/M0 (FIG. 2A). More upregulated proteins interacted with M1 macrophages than with M2 macrophages, but proteins present in the cell membrane did not interact. Among the membrane proteins, ITGB2 was found to be upregulated in M2 macrophages (FIG. 2B). In addition, cytokines such as IL-10 and CXCL10, known as markers of M1 macrophages, were upregulated in M1 macrophages (FIG. 2V). In addition, proteins were identified to be upregulated in M2 compared to M1, but among the membrane proteins, only ITGB2 was identified to be upregulated in M2 macrophages (FIG. 2D). Through this, it was confirmed that melittin interacted and bound with the membrane protein ITGB2 of M2 macrophages.

### 2-3. Identification of M2 TAM binding target protein of TAMpep826

As in Example 2-2 above, TAMpep826-biotin reacted with cell membrane proteins extracted from M0, M1, and M2 macrophages to identify target proteins through LC-MS/MS proteomics.

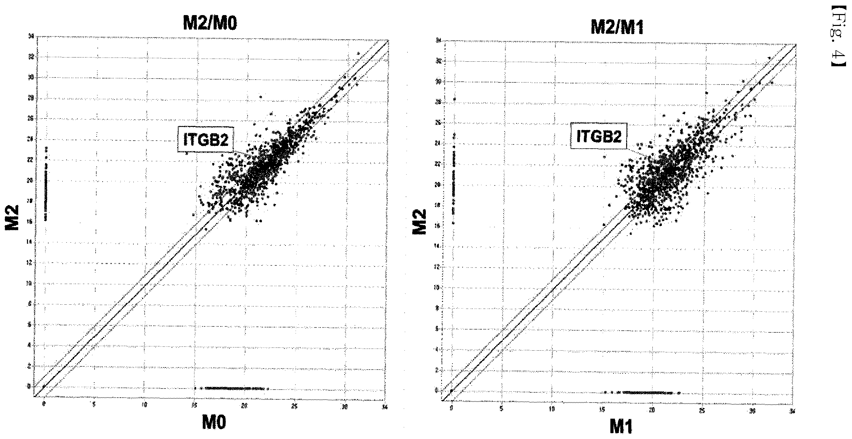

As a result, ITGB2, among the cell membrane proteins, was found to be expressed at a higher level in M2 macrophages than in M0 and M1 macrophages (FIG. 4), and was selected as a M2 macrophage binding target protein of TAMpep826.

### Example 3. Confirmation of ITGB2 expression in M2 macrophages

### 3-1. Confirmation of ITGB2 mRNA expression

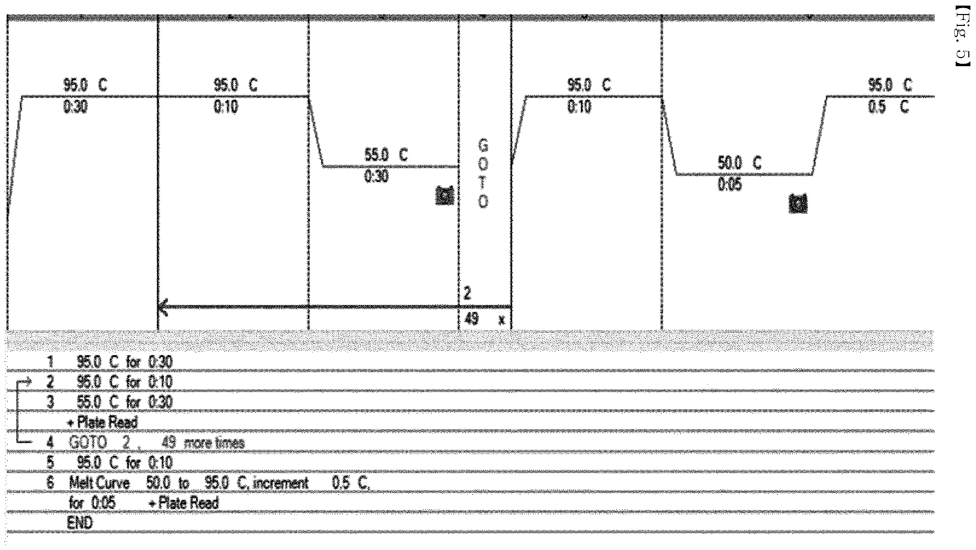

Real-time polymerase chain reaction was performed to evaluate the mRNA expression levels of ITGB2 in M0, M1, and M2 macrophages and TAMs. Specifically, RNA was extracted from cells differentiated into M0, M1, or M2 macrophages using an easy-BLUE total RNA Extraction Kit (iNtRON), and cDNA was synthesized using CycleScript-Reverse Transcriptase (Bioneer). Primers for an Actin or ITGB2 gene (Table 3) were used and real-time PCR (CFX96, Bio-rad) was performed under the conditions of FIG. 5 to compare and analyze the expression level of each gene relative to Actin through relative quantification.

**[Table 3]**

| Target cDNA | Forward/Reverse | Sequences (5' to 3') |
|---|---|---|
| β-Actin | F | GTGCTATGTTGCTCTAGACTTCG |
| | R | ATGCCACAGGATTCCATACC |
| ITGB2 | F | GAGGTCAGGAGGATTTGGGG |
| | R | TACAAGTGTTGGGGAGCCAG |

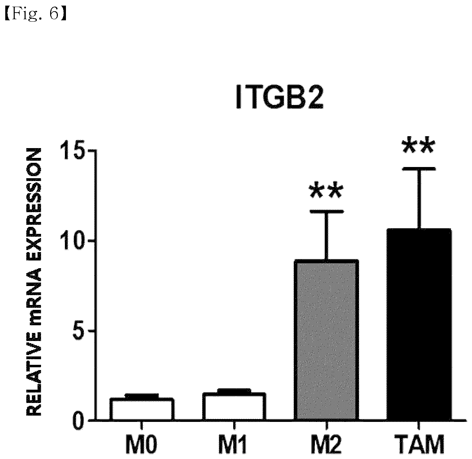

As a result, mRNA expression of ITGB2 was significantly increased in M2 macrophages and TAMs compared to M0 and M1 (FIG. 6).

### 3-2. Confirmation of ITGB2 protein expression

Western blot analysis was performed to evaluate the protein expression levels of ITGB2 in M0, M1, and M2 macrophages and TAMs. Specifically, proteins were extracted from each cell differentiated into M0, M1, and M2 macrophages using a Proprep solution (iNtRON), and the proteins were transferred to the membrane by electrophoresis. The membrane was blocked in 5% BSA, treated with a primary antibody (ITGB2) for 24 hours at 4°C, treated with a secondary antibody for 1 hour at room temperature, and then treated with the D-Plus^{™} ECL Pico System (Dong-In LS), and protein bands were photographed using the Davinch-Chemi^{™} imaging system (Intoxia).

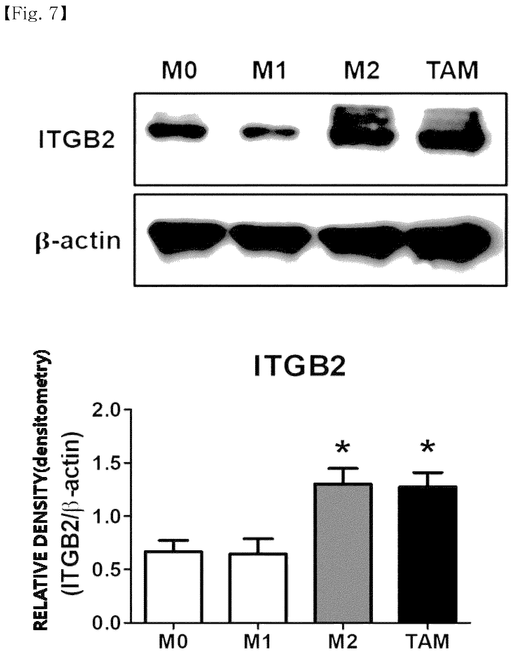

As a result, it was shown that the protein expression of ITGB2 was significantly increased in M2 macrophages and TAMs compared to M0 and M1 (FIG. 7).

### 3-3. Confirmation of ITGB2 protein expression by immunofluorescence

Immunofluorescence analysis was performed to evaluate the protein expression levels of ITGB2 in M0, M1, and M2 macrophages and TAMs. Specifically, each cell differentiated into M0, M1, and M2 macrophages was fixed with 4% formalin, washed with PBS, blocked in 5% BSA, and then treated with a primary antibody (ITGB2) for 24 hours at 4°C and a secondary antibody for 2 hours at room temperature and stained. After DAPI mounting, the stained cells were photographed with fluorescence using LSM800 (Zeiss).

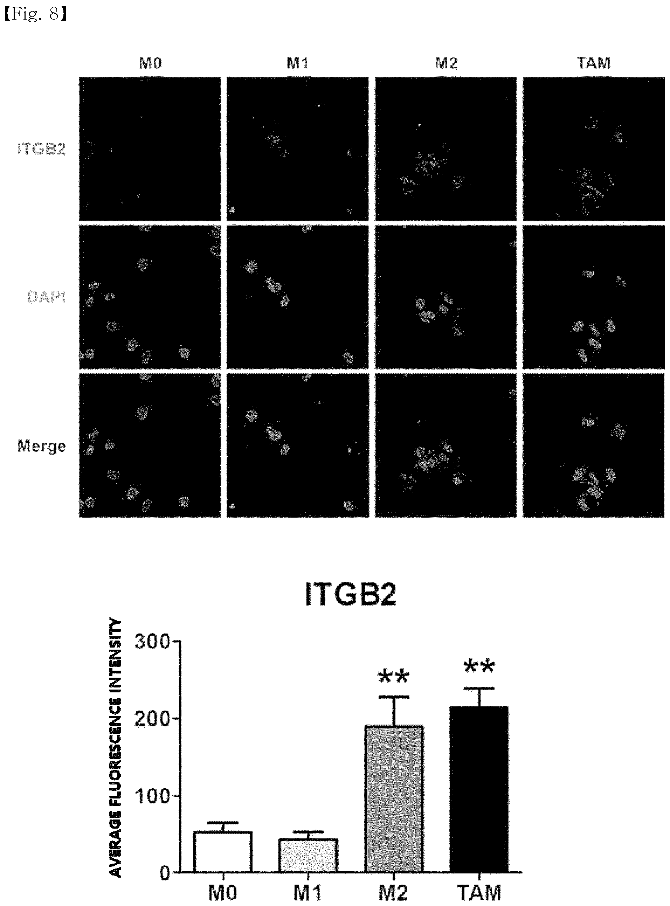

As a result, the protein of ITGB2 showed higher expression levels in M2 macrophages and TAMs compared to M0 and M1 (FIG. 8).

### Example 4. Confirmation of interaction between M2 TAM targeting peptide and ITGB2

### 4-1. Confirmation of ITGB2 expression on M2 macrophage cell membrane

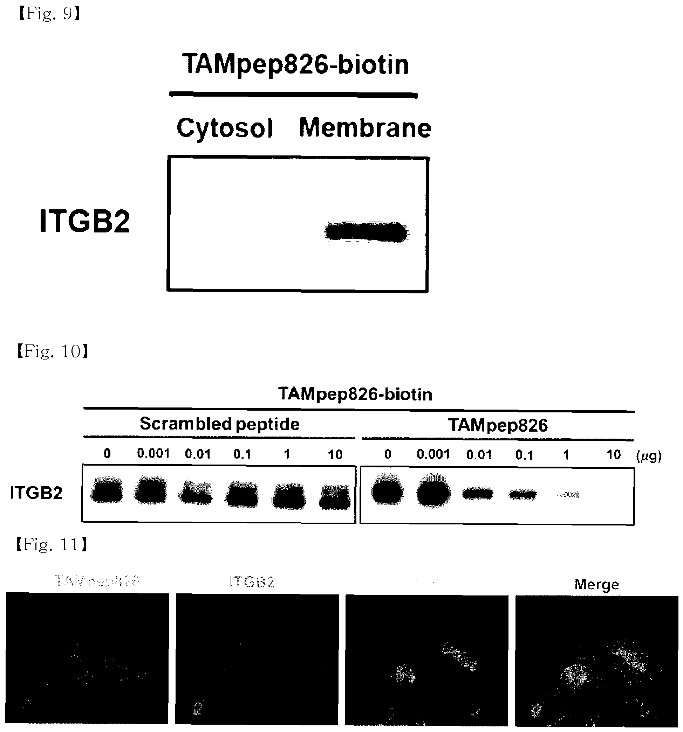

To determine whether ITGB2 was expressed on the cell membrane of M2 macrophages, cell membrane proteins and cytosol proteins were isolated and extracted from M2 macrophages, respectively, and reacted with biotinylated TAMpep826 to evaluate the expression of ITGB2 by Western blot analysis. As a result, it was shown that the ITGB2 protein was expressed on the cell membrane of M2 macrophages (FIG. 9).

### 4-2. Confirmation of interaction between TAMpep826 and ITGB2

To determine whether TAMpep826 interacted with ITGB2 in M2 macrophages, the expression of ITGB2 was evaluated through competitive reaction of TAMpep826 and biotinylated TAMpep826. Specifically, cells differentiated into M2 macrophages were collected with a scraper, the cells were washed by centrifugation (300 × g, 5 min), and the cell membrane proteins of macrophages were obtained using a cell membrane protein extraction kit (Thermo Fisher scientific). After pretreatment with TAMpep826 or scramble peptide for each concentration (0.001, 0.01, 0.1, 1, and 10 µg) for 1 hour, the proteins reacted with biotin-conjugated TAMpep826 (200 µg) at room temperature (20 to 24°C) for 1 hour, and then the proteins bound to TAMpep826 were obtained using Dynabeads^{™} M-280 Streptavidin (Thermo Fisher scientific). The expression of ITGB2 was confirmed by Western blot analysis of the proteins.

As a result, when pretreated with the scrambled peptide, the expression of ITGB2 bound to biotinylated TAMpep826 was not affected, whereas when pretreated with TAMpep826, ITGB2 expression was suppressed depending on a concentration (FIG. 10). Through this, it can be confirmed that TAMpep826 interacts with ITGB2 on the cell membrane of M2 macrophages.

### 4-3. Confirmation of interaction between TAMpep826 and ITGB2 using immunofluorescence

To determine whether ITGB2 and TAMpep826 were co-localized on the cell membrane of M2 macrophages, immunofluorescence analysis was performed using FITC-conjugated TAMpep826. As a result, it was shown that TAMpep826 and ITGB2 were intensively distributed in the cell membrane of M2 macrophages (FIG. 11).

### 4-4. Identification of binding sites of TB511 and ITGB2

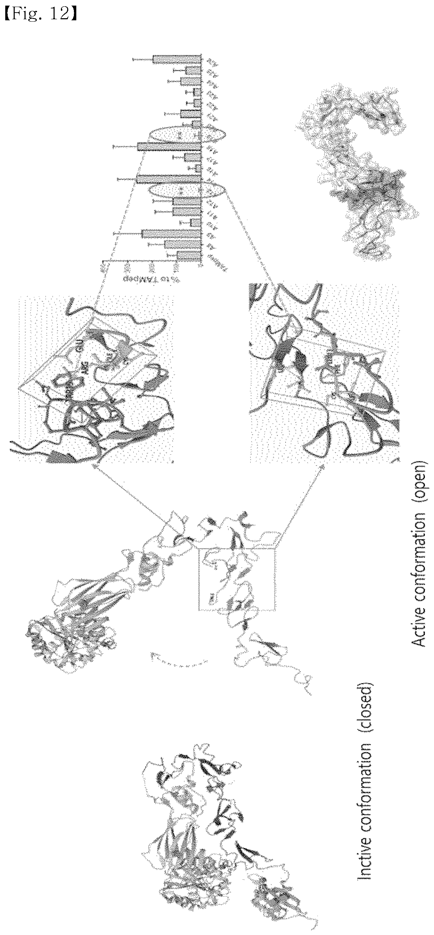

Based on a sequence of a cysteine rich region of CD18 (ITGB2) (uniport_p05107, Cystein-rich tandem repeats region: 449-617), a tertiary structure was constructed using a trRosetta algorithm (https://yanglab.nankai.edu.cn/trRosetta/). Thereafter, a docking simulation of TB511 and CD18 was performed using a CD18 PDB file created by trRosetta and an amino acid sequence of TB511 via a CABS-dock server (http://biocomp.chem.uw.edu.pl/CABSdock/). Among 10 models generated from the docking simulation result, a representative model was selected based on an alanine substitution result of the TB511 sequence (FIG. 12).

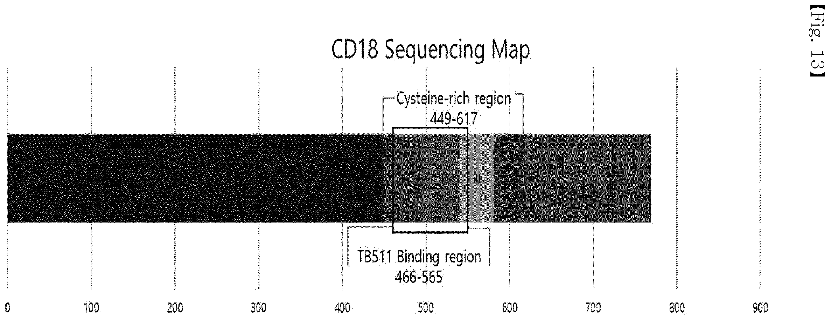

As a result, it was predicted that LEU(6) of TB511 bound to LEU(528), THR(538), LEU(556), CYS(557) and PHE(558) sites of CD18, and TRP(12) of TB511 bound to GLU(466), ILE(469), CYS(470) and ARG(471) of CD18. In addition, it was shown that when LEU(6) and TRP(12) of TB511 were each substituted with alanine to simulate binding to CD18, both the two regions did not bind to any sequence of CD18. Accordingly, LEU(6) and TRP(12) of TB511 are key parts in the docking between TB511 and CD18, and TB511 can be predicted to bind to positions 466 to 565 of CD18 (FIG. 13).

### Example 5. Synthesis of ITGB2 binding moieties

### 5-1. Synthesis of ITGB2-binding peptide-drug conjugate (PDC)

Melittin-dKLA and TB511 peptides in Table 4 below were synthesized and supplied by GenScript (Piscataway, NJ, USA) and were dissolved in D-PBS at 5 mg/ml and used in subsequent experiments. dKLA was linked through amide bonds between peptides, and a linker consisting of four glycines and one serine was placed in the middle to separate two ends so as to minimize the interaction and folding between melittin or TAMpep826 and dKLA. In addition, a D-enantiomer other than an L-enantiomer of KLA was used to minimize degradation in the body. In addition, in order to fabricate M-DM1, maleimide-modified melittin (GenScript, Beijing, China) (100 µM, 1 mL, 0.1 mmol) dissolved in 25 mM of a sodium borate buffer (25 mM NaCl, 1 mM EDTA, pH 8.0) was added with DM1 (Mertansine) (MedChem Express, Princeton) (1.1 equivalents, 10 mM in DMF) and dimethylformamide (DMF), and reacted at 37°C for 1 hour. Then, the product was filtered three times by ultrafiltration in Dulbecco's phosphate-buffered saline (DPBS; Welgene), and thus DM1 bound to the N-terminus of maleimide-modified melittin M to fabricate PDC, which was named as M-DM1. 5 µL of the fabricated M-DM1 was injected by reverse-phase high-performance liquid chromatography (HPLC) using a Poroshell 120 C18 column (2.7 µm, 3 × 50 mm, Agilent, Santa Clara, CA, USA) at a flow rate of 0.5 mL/min, and then the characteristics were analyzed.

**[Table 4]**

| Name | Sequence | SEQ ID NO: | Description |
|---|---|---|---|
| Melittin | GIGAVLKVLTTGLPALI SWIKRKRQQ | 1 | Melittin peptide |
| TAMpep826 | VLTTGLPALISWIKRKR QQ | 2 | Peptide in which first 7 amino acids of melittin are removed |
| Linker | GGGGS | 3 | |
| dKLA | d[KLAKLAKKLAKLAK] | 4 | Pro-apoptosis peptide |
| Melittin-dKLA | GIGAVLKVLTTGLPALI SWIKRKRQQ-GGGGS-d[KLAKLAKKLAKLAK] | 5 | Peptide with linker and drug dKLA attached to melittin |
| TB511 | VLTTGLPALISWIKRKR QQ-GGGGS-d[KLAKLAKKLAKLAK] | 6 | Peptide with linker and drug dKLA attached to TAMpep826 |
| M-DM1 | | | PDC conjugated with drug DM1 to maleimide-modified melittin |

### 5-2. Synthesis of ITGB2-binding antibody-drug conjugate (ADC)

### 5-2-1. ADC fabrication of anti-CD18 antibody and DM1

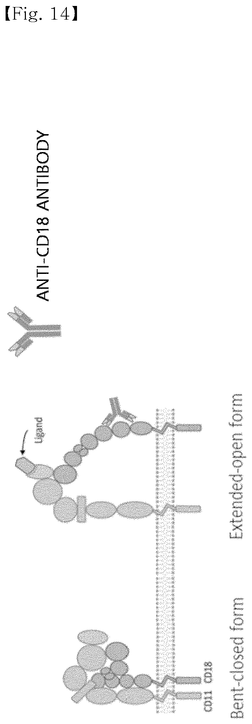

To fabricate an antibody that specifically bound to an active conformation of CD18, one of the macrophage-1 antigens (Mac-1), in M2 TAM (FIG. 14), a culture medium of hybridoma (MM18/2.a.8) cells secreting anti-CD18 antibodies was collected, centrifuged at 2,500 rpm and 4°C for 10 minutes to obtain a supernatant, and the supernatant was purified using protein G-Sepharose column chromatography. The antibody solution slowly passed through a protein G-Sepharose column (Pharmacia, Sweden) pre-equilibrated with PBS, and the column was connected to a peristatic pump and washed sufficiently with PBS. After washing, the antibody was eluted with 0.2 M glycine-HCl (pH 2.7). At this time, the eluate was buffered in a tube containing 1 M Tris (pH 9.0) prepared in advance. This antibody was used after dialysis in PBS. The amount of purified antibody was measured using a BCA^{™} protein assay kit (Pierce), and the avidity of the antibody was confirmed through flow cytometry. The purified anti-CD18 antibody was buffer-replaced with PBS (pH 7.4), and an anti-CD18 antibody (1 mg/ml, 6.67 µM) was mixed with SMCC-DM1 (mertansine, 120 µM) (MedChemExpress) dissolved in DMSO at a ratio of 1:18, and reacted at room temperature for 4 to 20 hours. To remove unreacted DM1, CD18-DM1 was buffer-replaced with PBS (pH 7.4) using a 10 KDa cutoff centrifugal filter, and filtered through a 0.2 µm syringe filter. The amount of DM1 (252 nm) bound to anti-CD18 antibody (280 nm) was measured using UV-Vis spectroscopy, and a drug antibody ratio (DAR) of DM1 to the anti-CD18 antibody was calculated, and the DAR value was 5.8. The fabricated ADC in which the anti-CD18 antibody and DM1 were bound was named as CD18-DM1.

### 5-2-2. ADC fabrication of anti-CD18 antibody and monomethyl auristatin E (MMAE)

In Example above, the purified anti-CD18 antibody was buffer-replaced with PBS (pH 7.4), and in order to partially reduce the disulfide bonds of the CD18 antibody, 1 mM DTT (66.7 µM) was mixed with an anti-CD18 antibody (1 mg/ml, 6.67 µM) at a ratio of 1:10 and reacted at 37°C for 1 to 2 hours, and then buffer-replaced with PBS (pH 7.4). The partially reduced anti-CD18 antibody (1 mg/ml, 6.67 µM) was mixed with 10 mM Suo-Val-Cit-PAB-MMAE (MedChemExpress) at a ratio of 1:20 to 1:40, reacted at 4°C for 12 hours, and buffer-replaced with PBS (pH 7.4). The amount of ve-MMAE (248 nm) bound to an anti-CD18 antibody (280 nm) was measured using UV-Vis spectroscopy, and a drug antibody ratio (DAR) of DM1 to the anti-CD18 antibody was calculated, and the DAR value was 2.8 to 5.4. The ADC fabricated by binding the anti-CD18 antibody and MMAE was named as CD18-MMAE.

### Example 6. Confirmation of M2 TAM binding to ITGB2 binding PDC

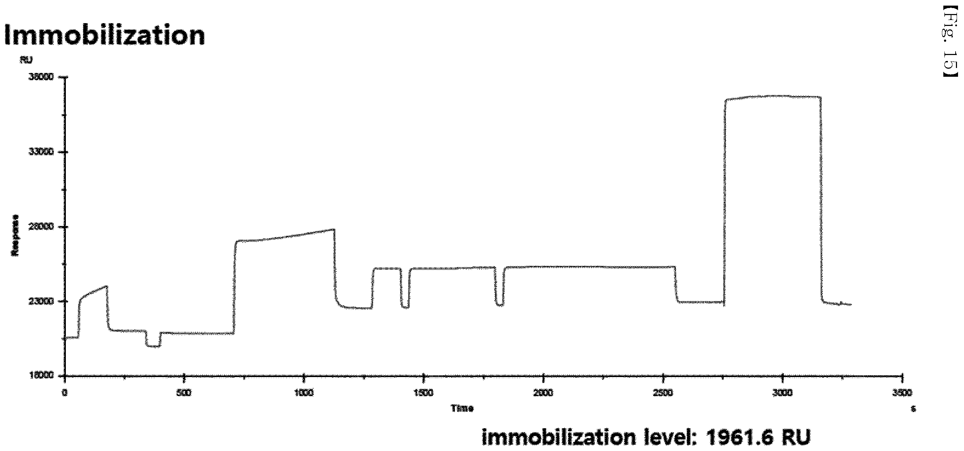

To confirm the binding affinity of ITGB2 and Melittin-dKLA, an ITGB2 protein was coated on a gold thin film sensor chip, and then ITGB2 antibody and Melittin-dKLA as positive controls were run, respectively, and the reflected light was measured and surface plasmon resonance (SPR) analysis was performed. Specifically, after ITGB2 was fixed on the sensor chip surface, immobilization was performed using amine coupling under conditions shown in Table 5 below (FIG. 15). Thereafter, various concentrations of ITGB2 antibody and Melittin-dKLA were run through the ITGB2-coated sensor chip to observe association and dissociation sections, and an intermolecular association rate constant (kinetic parameter) was calculated using a sensorgram under conditions of the intermolecular association rate constant analysis (kinetic evolution) in Table 6 below.

**[Table 5]**

| | | |
|---|---|---|
| coupling method | Amine coupling | |
| Sensor chip | HC1000M | |
| Running buffer | PBS pH 7.4 | |
| flow cell 1 | Activation | 200mM EDC, 100mM NHS |
| | Blocking protein | BSA |
| | Blocking | 1 M Ethanolamine*HCl |
| flow cell 2 | Activation | 200mM EDC, 100mM NHS |
| | Ligand concentration | 50 µg/ml in 5 mM acetate buffer (pH 4.0) |
| | Target RU | 5000 RU |
| | Blocking solution | 1 M Ethanolamine*HCl |

**[Table 6]**

| **2) Kinetic evaluation** | | | | |
|---|---|---|---|---|
| Running buffer | 1 × PBS | | | |
| analyte | name | Anti-ITGB2 antibody. TAMpep | | |
| | concentration | Anti-ITGB2 antibody | 1.5625. 3.125 6.25. 125.25, 50. 100n M | |
| | | TAMpep | 15.625. 31 25.62 5, 125, 250. 500, 100 0 nM | |
| Binding setting | Contact time (min) | 2 min | flow rate (µl/min) | 30 µl/min |
| | Dissociation time (min) | 5 min | Stabilization time ( min) | 0 |
| regeneration | Regeneration solution | Glycine pH 1.5 | flow rate (µl/min) | 30 µl/min |
| | Contact time (sec) | 45 sec | Stabilization time (min) | 2 min |

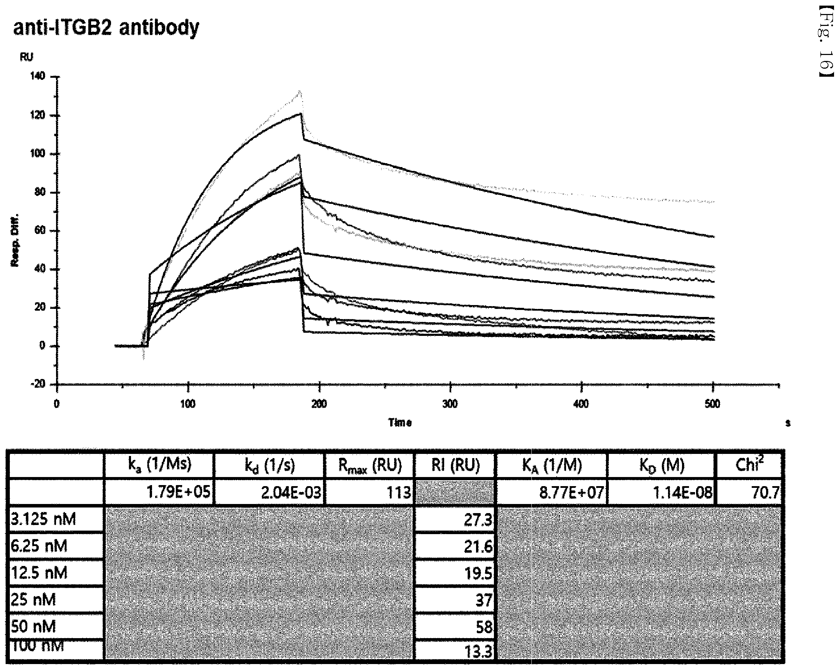

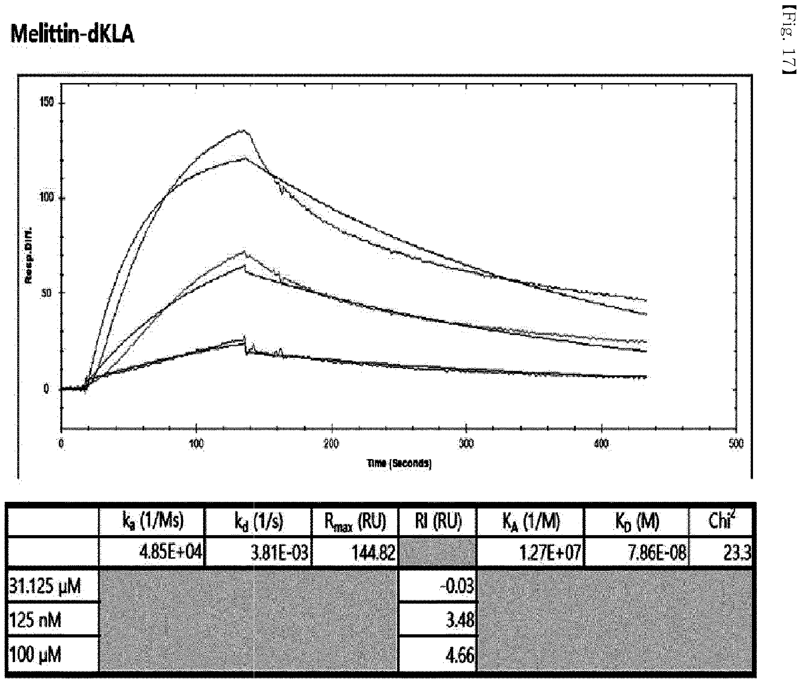

As a result, an equilibrium dissociation constant (KD), which was obtained by dividing a dissociation rate constant (kd) that evaluated the binding affinity between two molecules of a positive control ITGB2 antibody by an association rate constant (ka), was 1.14⁻⁸, and the equilibrium dissociation constant of Melittin-dKLA was 7.86⁻⁸ (FIGS. 16 and 17). Therefore, it was confirmed that Melittin-dKLA had binding affinity to ITGB2.

### Example 7. Confirmation of M2 TAM apoptotic effect through ITGB2 of PDC

### 7-1. Fabrication of cell model of ITGB2 expression suppression

As a cell model used to confirm whether apoptosis was induced through ITGB2 in M2 macrophages, a cell model in which the expression of ITGB2 was suppressed in M2 macrophages was fabricated using Crispr/cas9. Specifically, cells differentiated into M2 macrophages were cultured in an Opti-MEM medium. The Crispr/cas9 was mixed with Cas9 protein V2 (Thermo Fisher scientific) and gRNA (Genscript; ITGB2; Table 7) of a CRISPRMAX^{™} Reagent kit (Thermo Fisher scientific) and a Cas9 Plus^{™} Reagent (Thermo Fisher scientific), and a CRISPRMAX^{™} Reagent was diluted in the Opti-MEM medium, mixed together, and reacted for 5 to 10 minutes, and then added to cells differentiated into M2 macrophages and cultured at 37°C for 2 to 3 days. Thereafter, the mRNA expression level of ITGB2 was confirmed.

**[Table 7]**

| sgRNA | Sequences (5' to 3') |
|---|---|
| ITGB2 | mG*mU*mU*rCrArArCrGrUrGrArCrCrUrUrCrCrGrGrCrGrUrUrUrUrA rGrArGrCrUrArGrArArArUrArGrCrArArGrUrUrArArArArUrArArGrGr CrUrArGrUrCrCrGrUrUrArUrCrArArCrUrUrGrArArArArArGrUrGrGrC rArCrCrGrArGrUrCrGrGrUrGrCrU*mU*mU* mU |

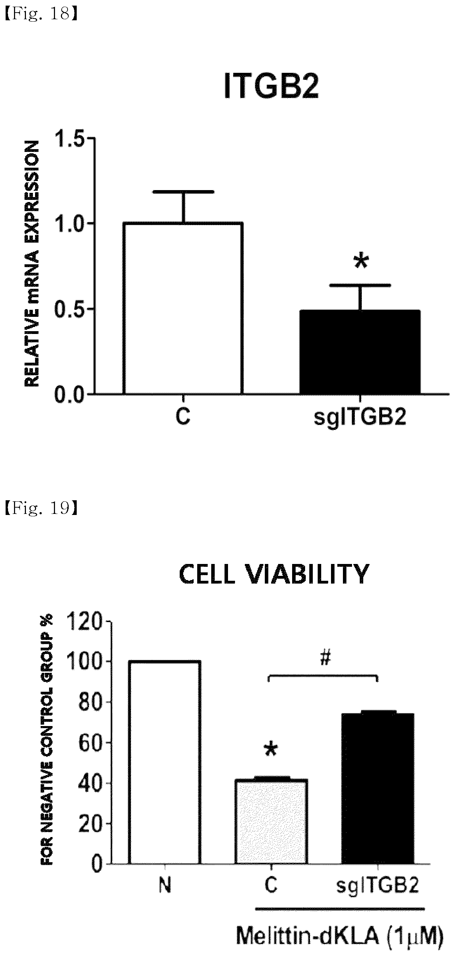

As a result, it was shown that in M2 macrophages injected with ITGB2 sgRNA, ITGB2 expression was significantly decreased compared to the control group (FIG. 18).

### 7-2. Confirmation of M2 TAM apoptosis through ITGB2 of Melittin-dKLA

To confirm whether Melittin-dKLA bound to ITGB2 to induce apoptosis in M2 macrophages, Melittin-dKLA (1 µM) was treated for 24 hours to M2 macrophages in which ITGB2 expression was suppressed by Crispr/cas9 fabricated in Example 7-1 above, and cell viability was analyzed using a CCK-8 assay. Specifically, Melittin-dKLA (1 µM) reacted with ITGB2 knockdown macrophages fabricated in Example 7-1 above for 1 hour. After the reaction, the medium was replaced and cultured at 37°C for 24 hours. To determine cell viability, a CCK-8 reagent (Enzo Life Sciences) was added in 1/10 of the medium and reacted at 37°C for 3 hours. Absorbance was measured at 450 nm using a microplate reader (Molecular Devices).

As a result, in control M2 macrophages, cell viability was significantly reduced by Melittin-dKLA, but in M2 macrophages in which ITGB2 was knocked down, the decrease in cell viability by Melittin-dKLA was suppressed (FIG. 19). Therefore, through this, it could be confirmed that Melittin-dKLA induced apoptosis of M2 macrophages by specifically binding to ITGB2 of M2 macrophages.

### 7-3. Confirmation of M2 TAM apoptosis through ITGB2 of TB511

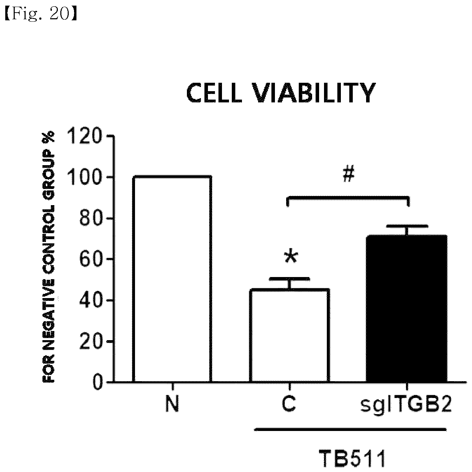

To confirm whether TB511 (TAMpep826-dKLA) induced apoptosis through ITGB2 in M2 macrophages, TB511 reacted with M2 macrophages in which ITGB2 expression was suppressed by Crispr/cas9 fabricated in Example 7-1. As a result, the M2 macrophages showed about 50% of cell viability by TB511, but the cell viability significantly increased in cells where ITGB2 expression was suppressed (FIG. 20).

### 7-4. Confirmation of reduced apoptosis induction of TB511 by ITGB2 antibody

To confirm whether TB511 induced apoptosis through ITGB2 in M2 macrophages, the M2 macrophages with suppressed ITGB2 expression fabricated in Example 7-1 were pretreated with an anti-ITGB2 antibody (Polyclonal Rabbit antiHuman ITGB2/CD18 antibody) (LS-C312785; LS Bio) (1 µg) for 1 hour, and then reacted with TB511 (1 µM) for 1 hour. Thereafter, the induction of apoptosis was confirmed by cell viability analysis using the CCK-8 assay, protein expression of caspase-3, an apoptosis marker, was confirmed by Western blot analysis, gene expression of caspase-3, caspase-8, and caspase-9 was confirmed by Real-time PCR, and induction of protein expression of caspase-3 was confirmed by immunofluorescence.

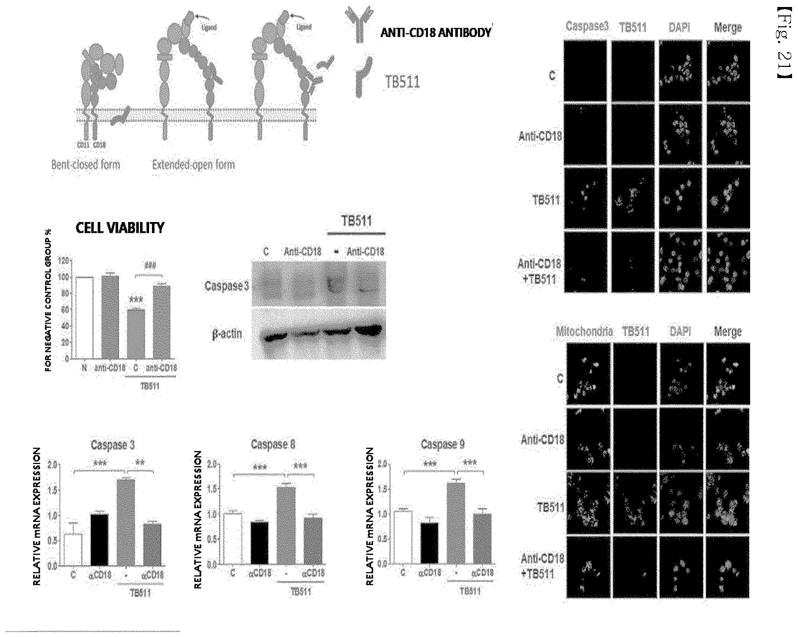

As a result, the M2 macrophages showed no effect on cell viability by the ITGB2 antibody, but cell viability was significantly reduced by TB511. On the other hand, in M2 macrophages pretreated with the ITGB2 antibody, the cell viability was significantly increased by TB511 (FIG. 21). In addition, caspase-3 expression was significantly increased by TB511 in M2 macrophages, whereas the caspase-3 expression was significantly decreased by TB511 in M2 macrophages pretreated with the ITGB2 antibody (FIG. 21). In addition, in M2 macrophages, the expression of caspase-3, caspase-8, and caspase-9 was significantly increased by TB511, whereas in M2 macrophages pretreated with the ITGB2 antibody, the expression of caspase-3, caspase-8, and caspase-9 was significantly decreased by TB511 (FIG. 21). In addition, in M2 macrophages, the caspase-3 expression was increased by TB511, whereas in M2 macrophages pretreated with the ITGB2 antibody, the caspase-3 expression was decreased by TB511 (FIG. 21). Additionally, as a result of confirming the mitochondrial targeting of TB511, in the M2 macrophages pretreated with ITGB2 antibody, co-localization of the mitochondria and TB511 was decreased compared to cells not pretreated with ITGB2 (FIG. 21).

### Example 8. Confirmation of efficacy of PDC in 3D cancer organoids

### 8-1. Confirmation of effect of TB511 in breast cancer 3D organoids

To complement the limitations in vitro and replace in vivo, a 3D organoid mimicking a tumor microenvironment (TME) of breast cancer was fabricated and an effect of TB511 thereon was confirmed. Specifically, an RPMI-1640 culture medium was mixed to contain 3% Matrigel, and MDA-MB-231 human breast cancer cells, CAFs (Cancer-associated Fibroblasts), and THP-1 cells fabricated to express GFP using MDA-MB-231 human breast chancer cells, Cancer-associated Fibroblast (CAF), and Incucyte^{®}Nuclight Lentivirus Reagents (Sartorius) were counted and dispensed into a 96 u-bottom 3-D culture plate (Sbio) at a ratio of 5:1:1 to be 200 µl. After culturing, the cells were collected by centrifugation at 1200 rpm for 3 minutes and then cultured in a 37°C incubator for 48 hours to form spheroids. From day 2 of spheroid formation, TB511 was treated every three days at concentrations of 1 µM, 2 µM, 4 µM, and 8 µM, and images were taken using a fluorescence microscope. After taking bright-field images, the spheroid area was measured using a method for measuring a Ferret diameter. In addition, the average tumor spheroid diameter was measured using an open source, and the average diameter of three spheroids was analyzed with ImageJ (software version 1.47 m). The spheroid size was analyzed by taking bright-field images (20X), and then measuring the average tumor spheroid diameter of three spheroids with ImageJ (software version 1.47 m). Additionally, on day 10 of drug treatment, immunofluorescent staining was performed by fixing spheroids and reacting with an antibody (abcam) against ki-67, a tumor proliferation factor (control staining: DAPI). For THP-1, images of GFP fluorescence expressed within the cells were taken using a confocal microscope without performing separate staining, and the number of cells expressing fluorescence was measured.

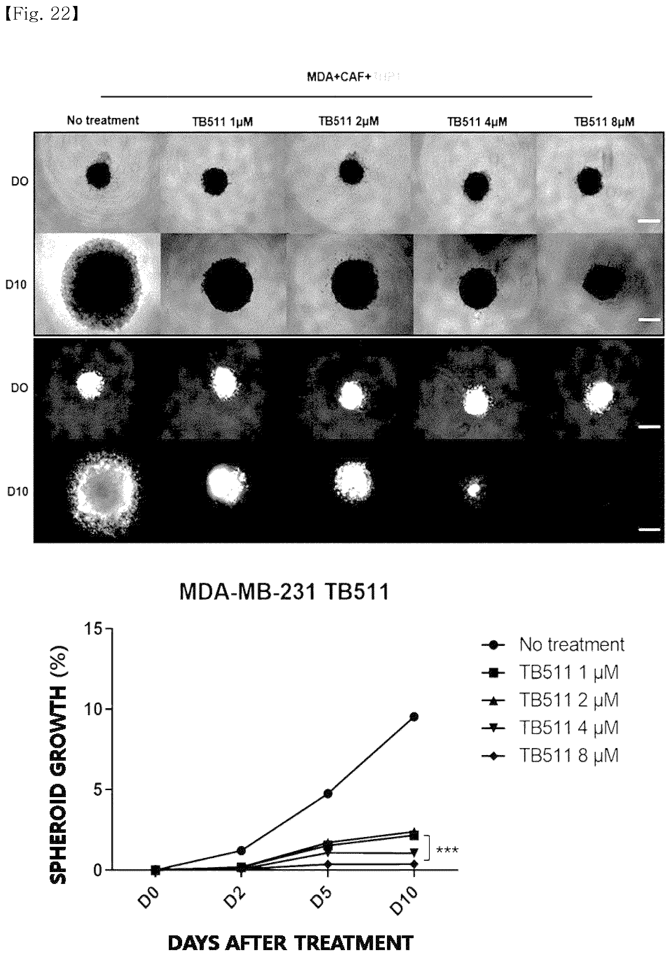

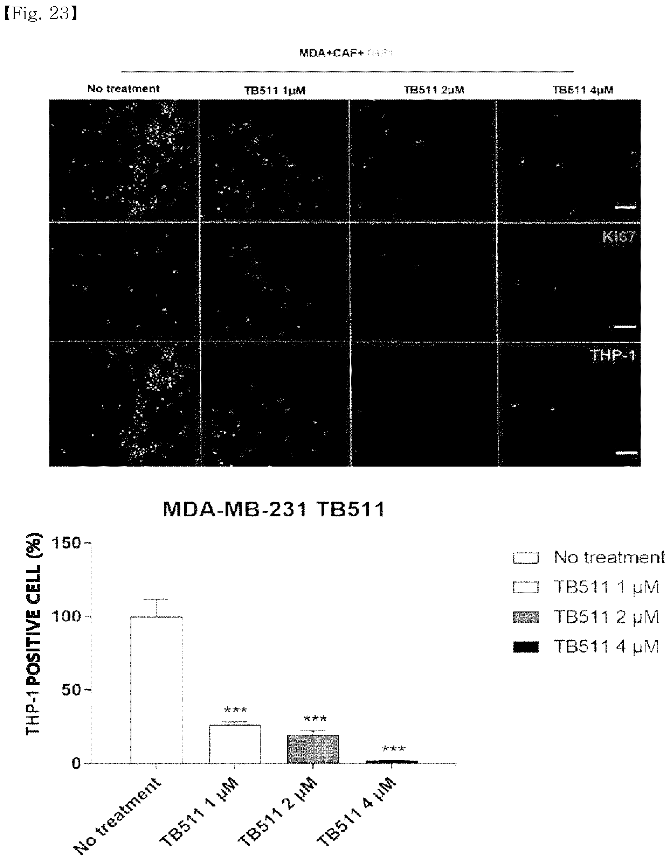

As a result, it was shown that in a group that was not treated with a drug, the size of the spheroids gradually increased over time, and thus THP-1 cells, which were monocytes, differentiated into TAMs by cancer cells and TME when co-cultured with cancer cells and CAFs, thereby promoting tumor growth. On the other hand, in a group treated with TB511, it was confirmed that the size of spheroids decreased in a concentration-dependent manner from a concentration of 1 µM on day 10 (FIG. 22), and it was shown that the number of THP-1 cells expressing GFP fluorescence was significantly decreased in a concentration-dependent manner compared to a drug-untreated group (***p < 0.001) (FIG. 23). Thus, it was confirmed that TB511, an ITGB2-binding PDC, selectively bound to THP-1 cells and induced apoptosis of MDA-MB-231 human breast cancer.

### 8-2. Confirmation of effect of TB511 in lung cancer 3D organoids

Lung cancer 3D spheroids were fabricated by mixing A549 human lung cancer cells, CAFs, and THP-1 cells using the method of Example 8-1 above, and then treated with TB511 at concentrations of 1 µM, 2 µM, and 4 µM, respectively, and the effects thereof were analyzed as in Example 8-1 above. When fabricating the spheroids, 3D spheroids were also fabricated by mixing cancer cells alone or only cancer cells + CAF cells, and the sizes of the spheroids were compared.

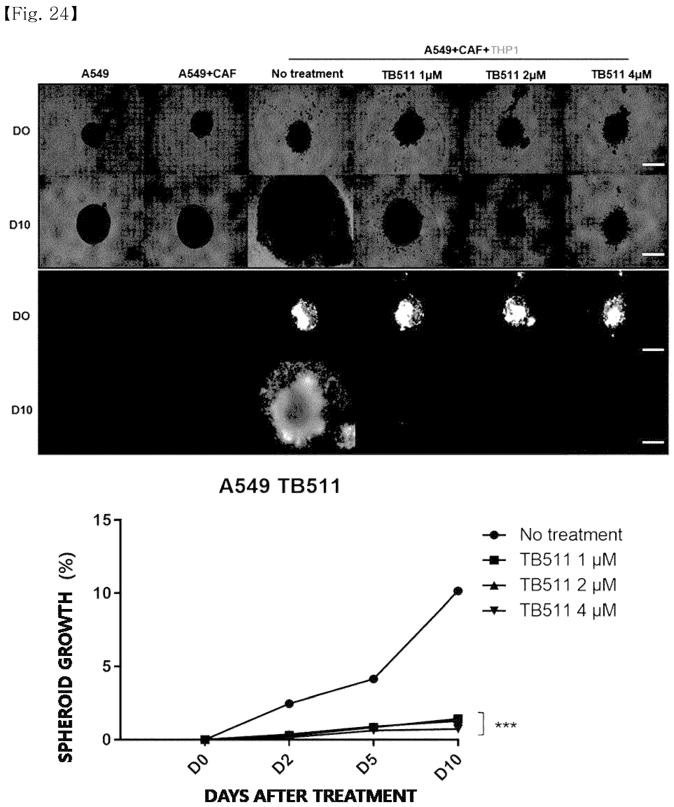

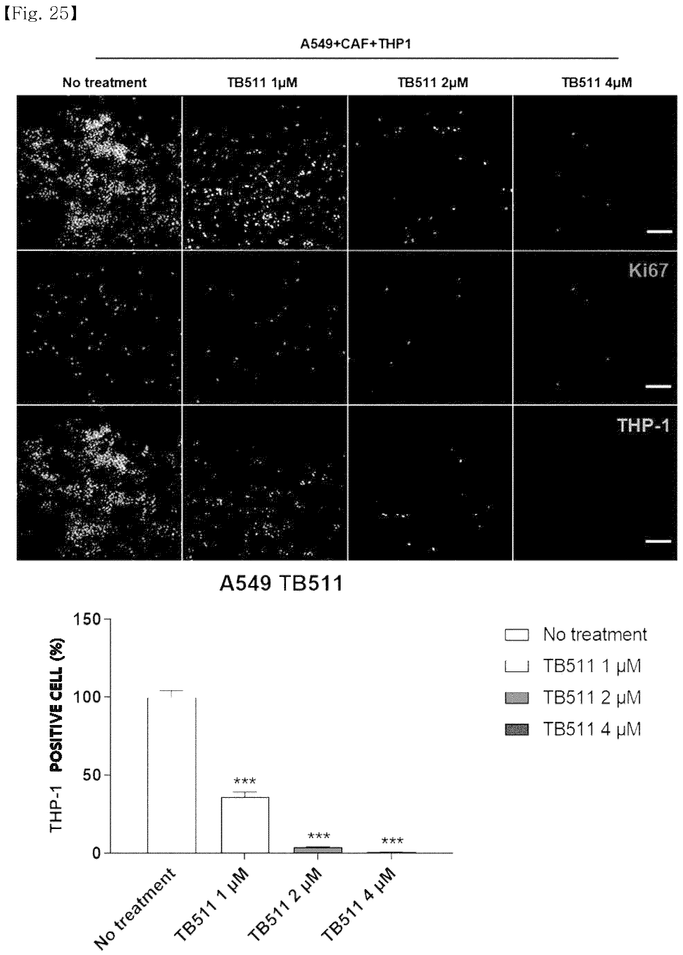

As a result, it was shown that the size of the spheroids was significantly increased when spheroids were formed with A549+CAF+THP-1 cells compared to when spheroids were formed with A549 cells alone or only A549 + CAF cells. In addition, the size of spheroids in a TB511-treated group was significantly reduced in a concentration-dependent manner compared to a group not treated with TB511 (***p < 0.001) (FIG. 24). In addition, as a result of confirming related markers by fluorescently staining the spheroids on day 10 after drug treatment, compared to the group not treated with TB511, the number of THP-1 cells was significantly decreased in a concentration-dependent manner, and a tumor proliferation factor ki-67 was also decreased in a concentration-dependent manner (***p < 0.001) (FIG. 25).

### 8-3. Confirmation of effect of M-DM1 in lung cancer 3D organoids

In order to confirm the efficacy of M-DM1 (Mel-DM1) on lung cancer, lung cancer 3D spheroids were fabricated by mixing A549 human lung cancer cells, CAFs, and THP-1 cells using the method of Example 8-1 above, and then treated with M-DM1 at concentrations of 1 µM, 2 µM, and 4 µM, respectively, and the effects thereof were analyzed as in Example 8-1 above.

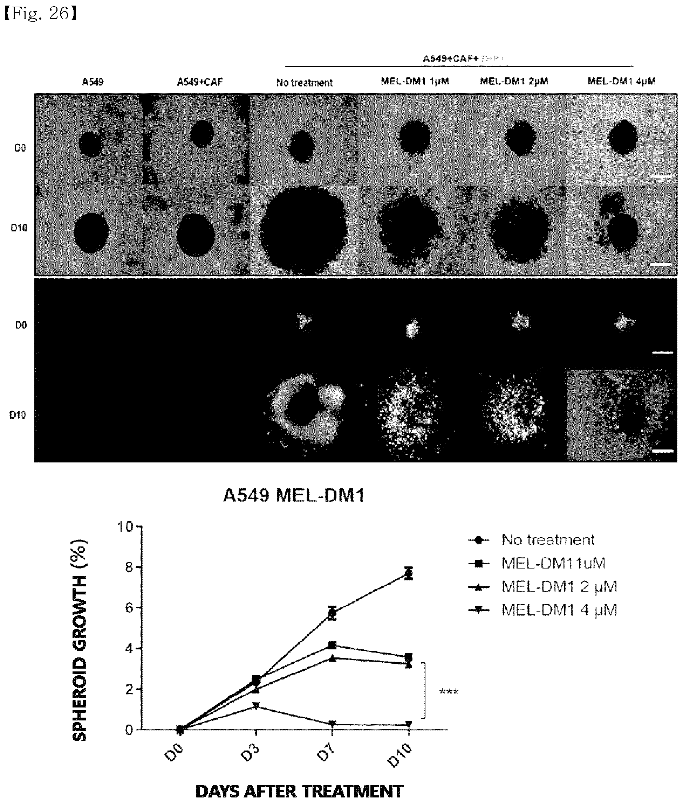

As a result, it was shown that the size of spheroids was decreased in a concentration-dependent manner in a group treated with M-DM1 compared to a group not treated with M-DM1 (***p < 0.001) (FIG. 26).

### Example 9. Confirmation of efficacy of PDC in anticancer drug-resistant 3D cancer organoids

### 9-1. Confirmation of effect of TB511 in anticancer drug-resistant lung cancer 3D organoids

In order to confirm the efficacy of TB511 on Carboplatin-resistant lung cancer, 3D spheroids were formed by mixing Carboplatin-resistant A549 cells, CAFs, and THP-1 cells using the method of Example 8-1 above, and then treated with TB511 at concentrations of 1 µM, 2 µM, and 4 µM, respectively, and the effects thereof were analyzed as in Example 8-1 above.

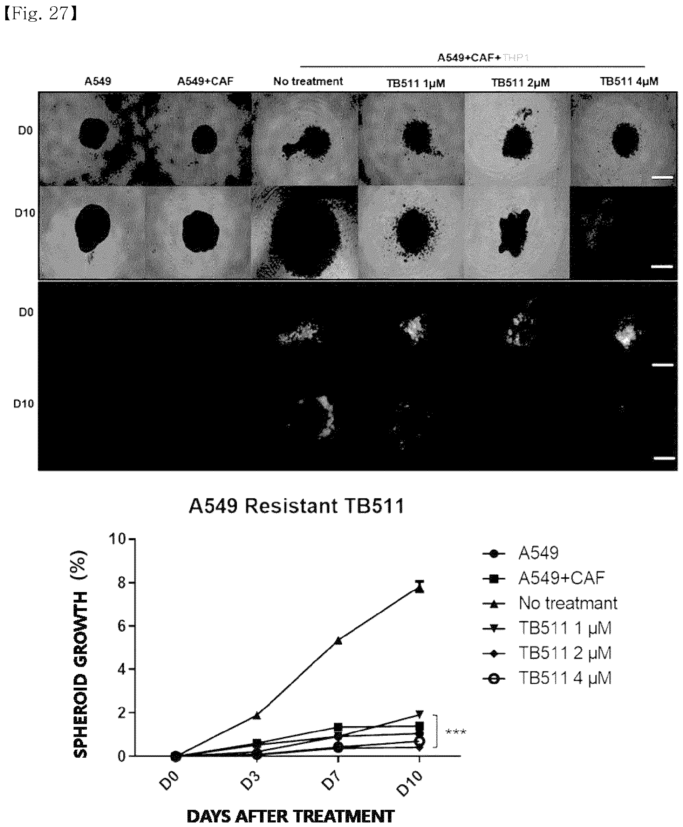

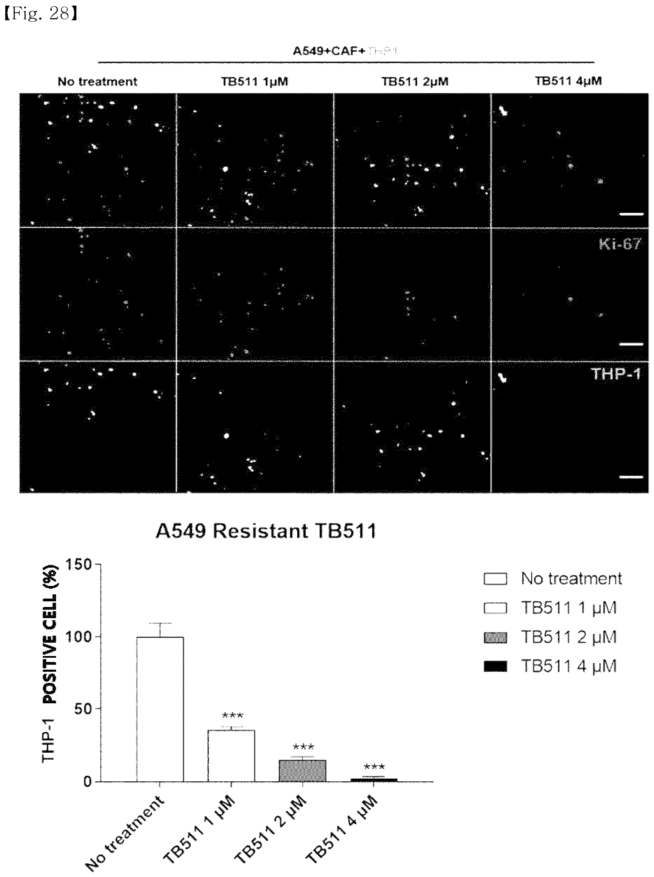

As a result, the size of the spheroids when the spheroids were formed with A549+CAF+THP-1 cells was significantly increased compared to when the spheroids were formed with A549-resistant cells alone or only A549+CAF cells, and the size of the spheroids in a TB511-treated group was significantly reduced in a TB511 concentration-dependent manner compared to a group not treated with TB511 (***p < 0.001) (FIG. 27). In addition, as a result of confirming related markers by staining the spheroids with immunofluorescence on day 10 after drug treatment, the number of THP-1 cells in the TB511-treated group was significantly decreased in a concentration-dependent manner compared to the TB511-untreated group, and the tumor proliferation factor ki-67 was also significantly decreased from 2 µM (***p < 0.001) (FIG. 28).

### 9-2. Confirmation of effect of TB511 in anticancer drug-resistant prostate cancer 3D organoids

In order to confirm the efficacy of TB511 on Docetaxel-resistant prostate cancer, 3D spheroids were formed by mixing Docetaxel-resistant PC3 prostate cancer cells, CAFs, and THP-1 cells using the method of Example 8-1 above, and then treated with TB511 at concentrations of 1 µM, 2 µM, and 4 µM, respectively, and the effects thereof were analyzed as in Example 8-1 above.

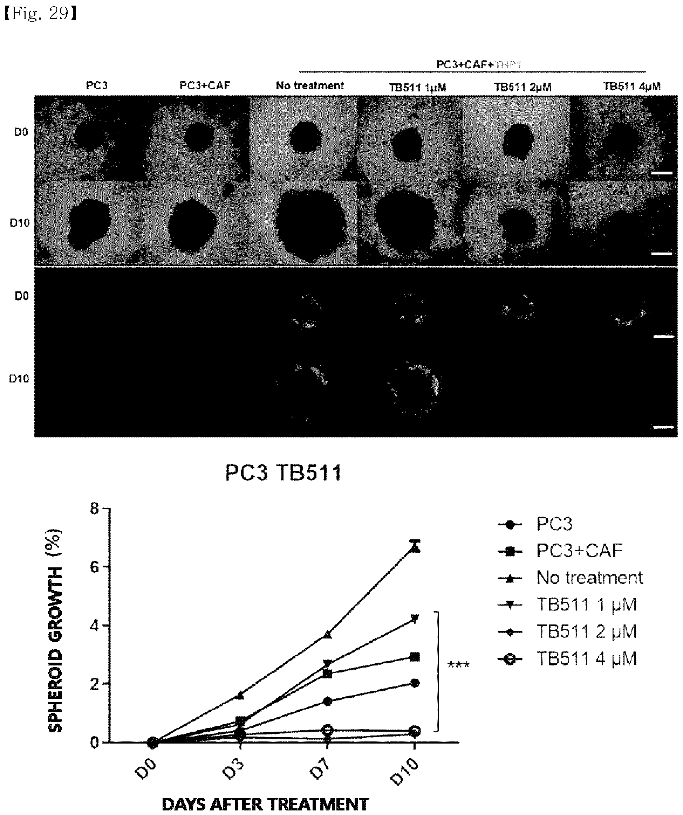

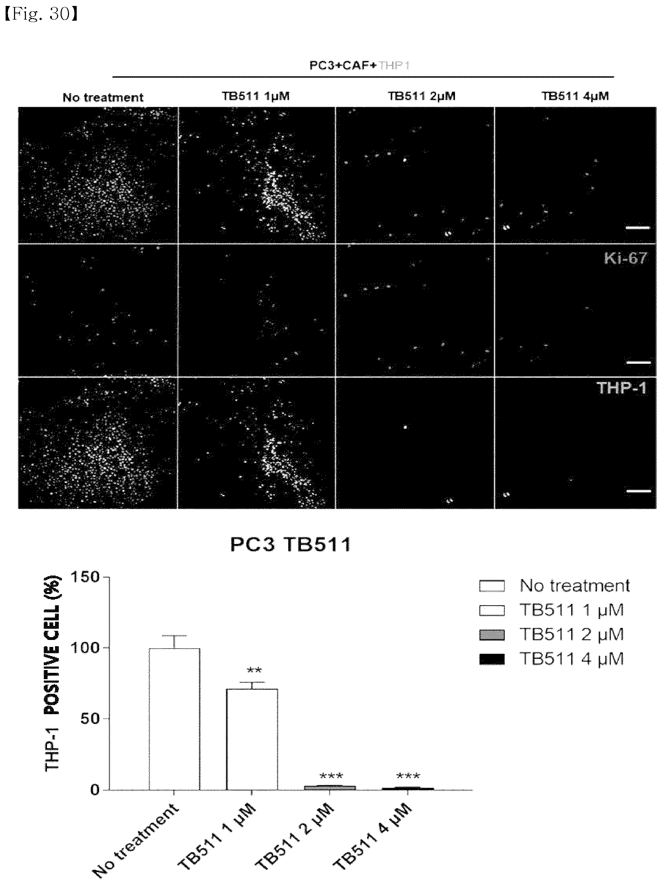

As a result, the size of the spheroids when the spheroids were formed with PC3+CAF+THP-1 cells was significantly increased compared to when the spheroids were formed with PC3-resistant cells alone or only PC3+CAF cells, and the size of the spheroids in a TB511-treated group was significantly reduced in a TB511 concentration-dependent manner compared to a group not treated with TB511 (***p < 0.001) (FIG. 29). In addition, as a result of confirming related markers by staining the spheroids with immunofluorescence on day 10 after drug treatment, the number of THP-1 cells in the TB511-treated group was significantly decreased in a concentration-dependent manner compared to the TB511-untreated group, and the tumor proliferation factor ki-67 tended to be reduced (**p < 0.01, ***p < 0.001) (FIG. 30).

### Example 10. Confirmation of efficacy of ADC in 3D cancer organoids

### 10-1. Confirmation of effect of ADC in breast cancer 3D organoids

In order to confirm the efficacy of an ITGB2-binding antibody-drug conjugate (ADC) on breast cancer, 3D spheroids were formed by mixing MDA-MB-231 human breast cancer cells, CAFs and THP-1 cells using the method of Example 8-1 above, and CD18-DM1 or CD18-MMAE, which was ADC of the present disclosure, was treated at concentrations of 10 µg and 20 µg, respectively, and the effects were analyzed as in Example 8-1 above.

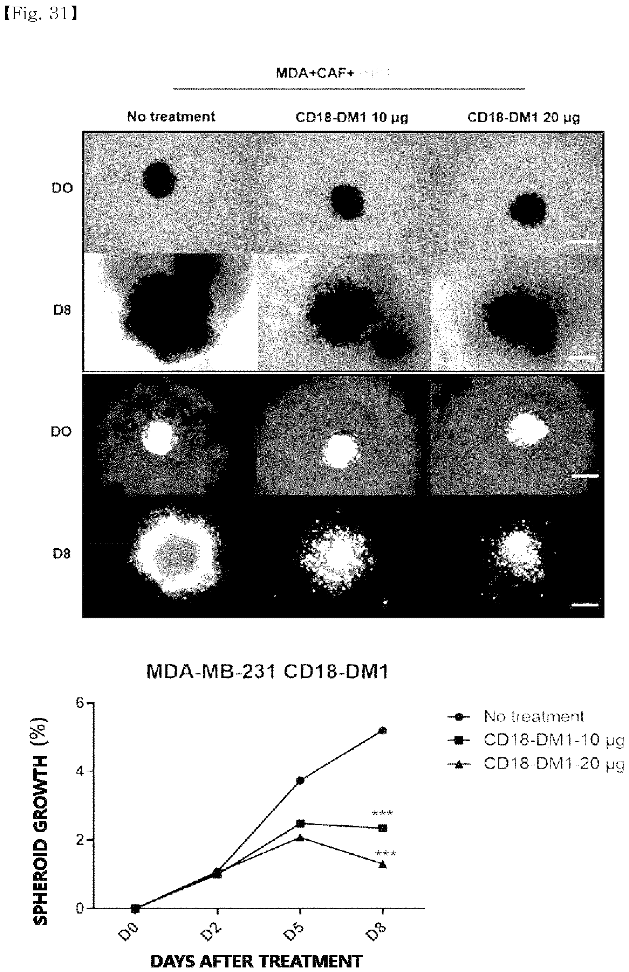

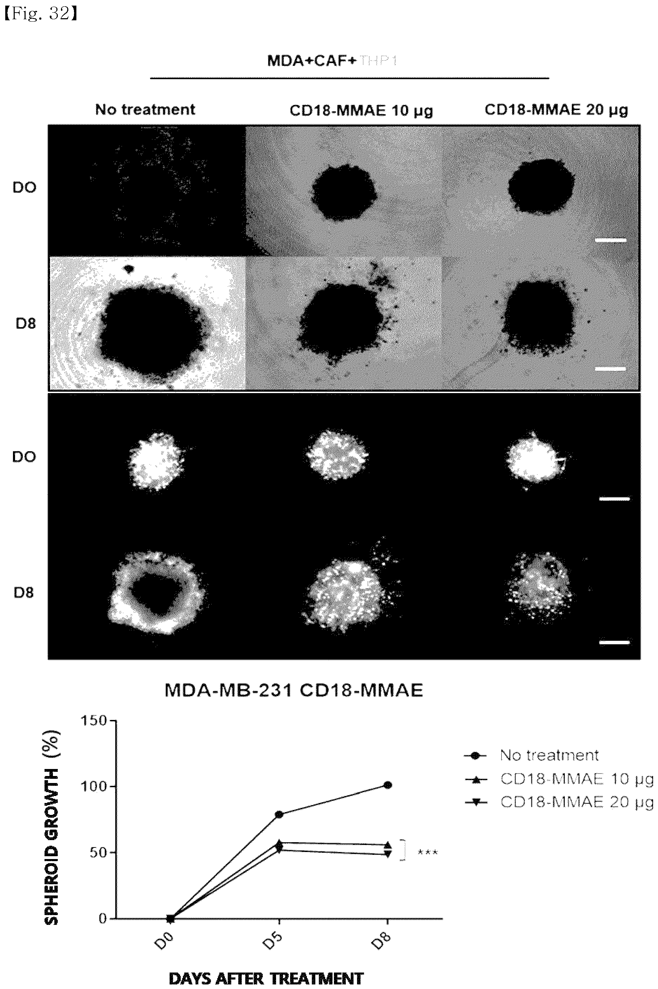

As a result, in a group not treated with ADC, the size of breast cancer spheroids was gradually increased over time, but in a group treated with ADC, the size of spheroids was decreased on day 8 (FIGS. 31 and 32). In addition, it was shown that GFP-positive THP-1 cells were strongly increased in the group not treated with ADC, but were decreased in a concentration-dependent manner in a CD18-DM1 or CD18-MMAE treated group (***p < 0.001) (FIGS. 31 and 32).

### 10-2. Confirmation of effect of ADC in lung cancer 3D organoids

In order to confirm the efficacy of an ADC on lung cancer, 3D spheroids were formed by mixing A549 human lung cancer cells, CAFs and THP-1 cells using the method of Example 8-1 above, and CD18-DM1 or CD18-MMAE, which was ADC of the present disclosure, was treated at a concentration of 10 µg, respectively, and the effects were analyzed as in Example 8-1 above.

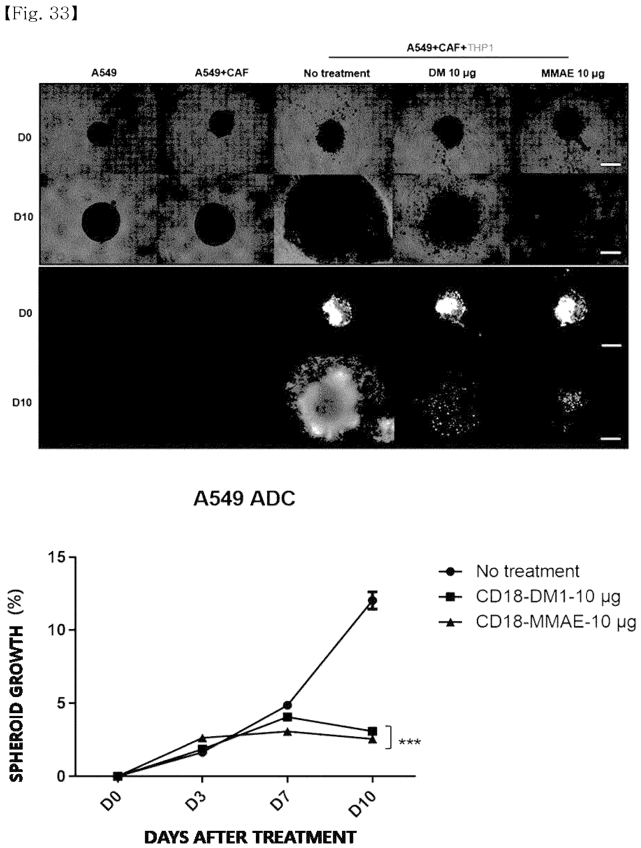

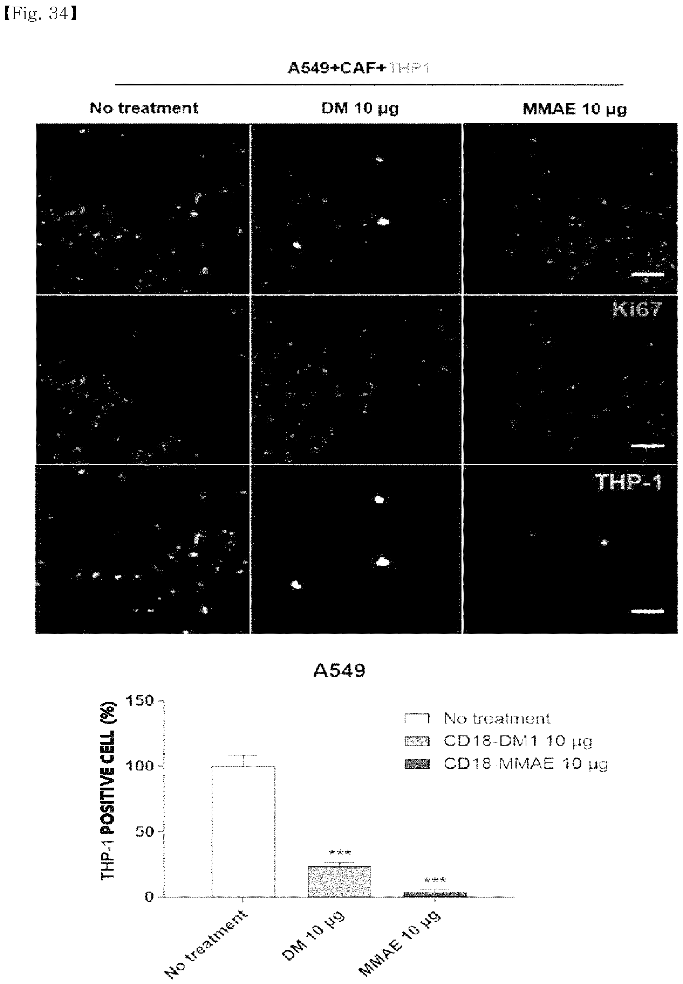

As a result, it was shown that in the group not treated with ADC, the size of spheroids was gradually increased over time, whereas in the group treated with CD18-DM1 or CD18-MMAE, the size of spheroids was decreased on day 8 (FIG. 33). In addition, as a result of confirming related markers by staining the spheroids with immunofluorescence on day 8 after drug treatment, it was found that THP-1 cells were strongly increased in the group not treated with ADC, whereas the THP-1 cells were significantly decreased in the ADC-treated group (***p < 0.001) (FIG. 34).

### Example 11. Confirmation of efficacy of ADC in anticancer drug-resistant 3D cancer organoids

### 11-1. Confirmation of effect of ADC in anticancer drug-resistant lung cancer 3D organoids

In order to confirm the efficacy of ADC on Carboplatin-resistant lung cancer, 3D spheroids were formed by mixing Carboplatin-resistant A549 cells, CAFs, and THP-1 cells using the method of Example 8-1 above, and then treated with CD18-DM1 or CD18-MMAE at a concentration of 10 µg, respectively, and the effects thereof were analyzed as in Example 8-1 above.

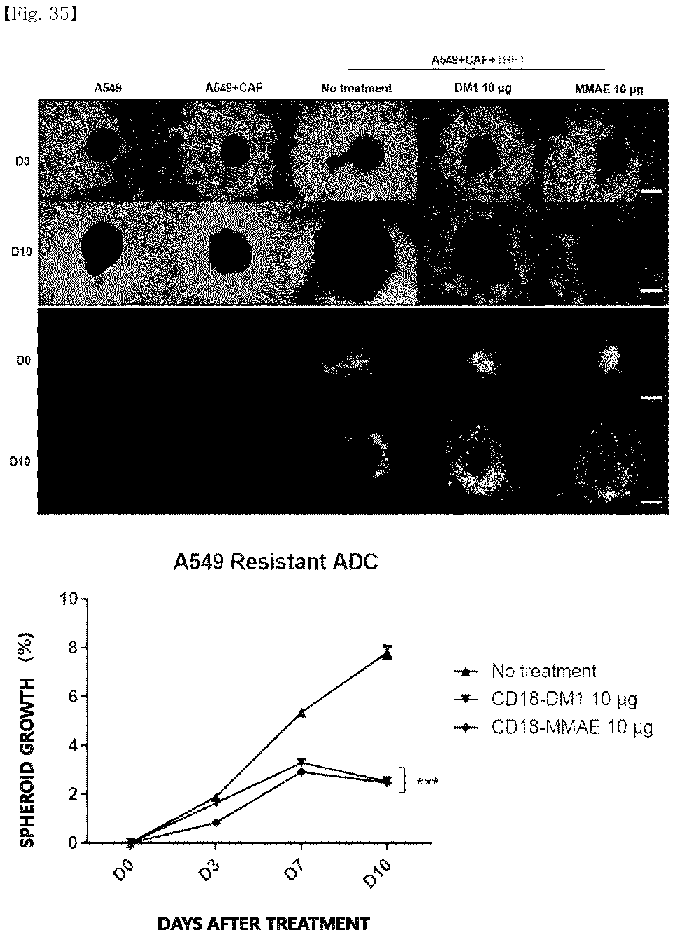

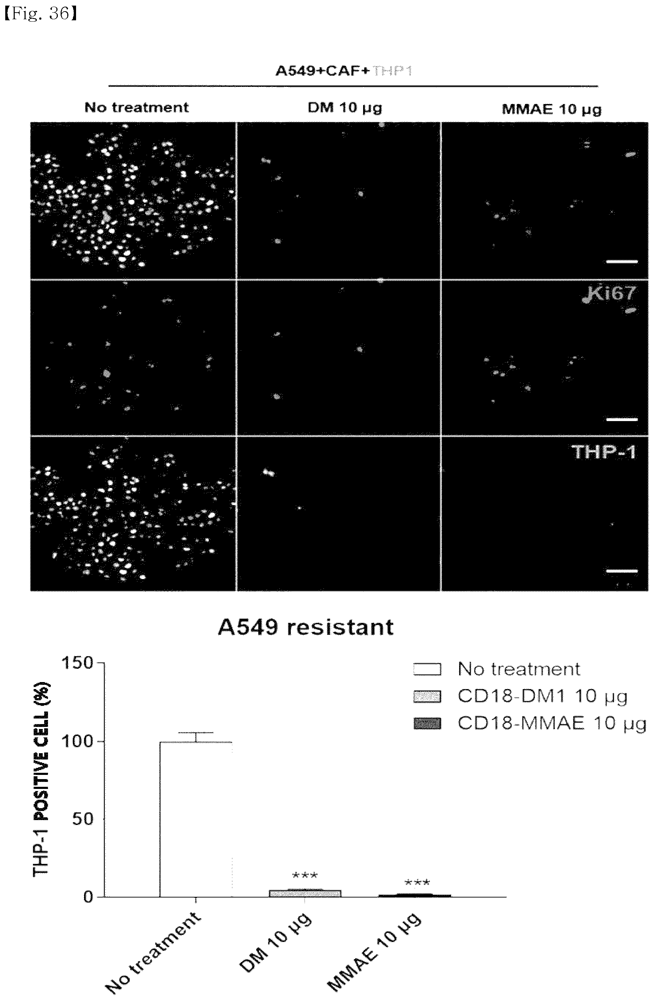

As a result, it was shown that in the group not treated with ADC, the size of spheroids was gradually increased over time, whereas in the group treated with CD18-DM1 or CD18-MMAE, the size of spheroids was decreased on day 8 (FIG. 35). In addition, as a result of confirming related markers by staining the spheroids with immunofluorescence on day 8 after drug treatment, it was shown that compared to the group not treated with ADC, in the group treated with CD18-DM1 or CD18-MMAE, the number of THP-1 cells was significantly decreased and the tumor proliferation factor ki-67 was also decreased (***p < 0.001) (FIG. 36).

### 11-2. Confirmation of effect of ADC in anticancer drug-resistant prostate cancer 3D organoids

In order to confirm the efficacy of ADC on Docetaxel-resistant prostate cancer, 3D spheroids were formed by mixing Docetaxel-resistant PC3 prostate cancer cells, CAFs, and THP-1 cells using the method of Example 8-1 above, and then treated with CD18-DM1 or CD18-MMAE at a concentration of 10 µg, respectively, and the effects thereof were analyzed as in Example 8-1 above.

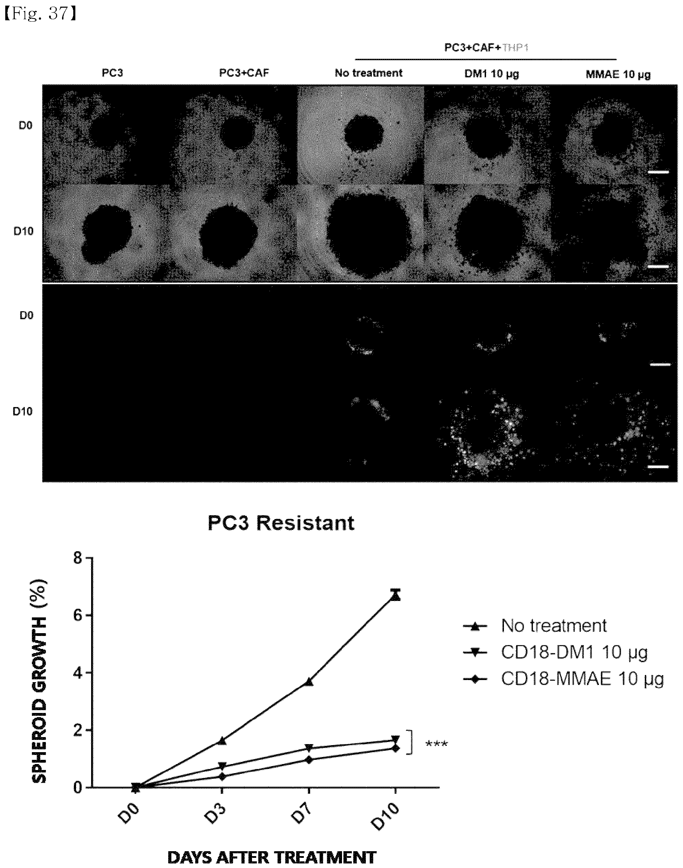

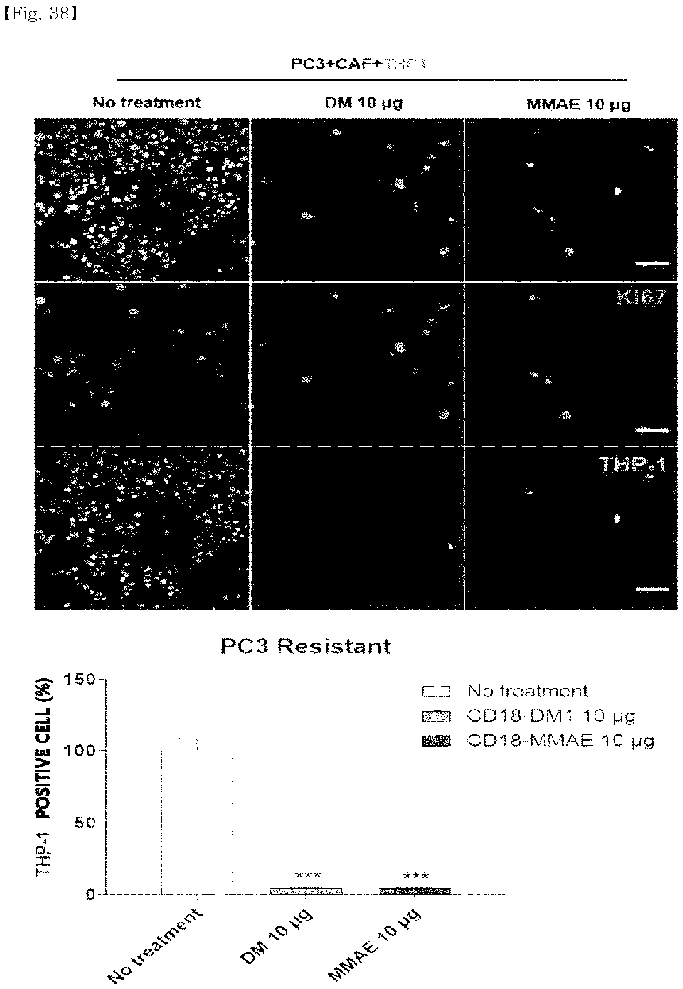

As a result, it was shown that in the group not treated with ADC, the size of spheroids was gradually increased over time, whereas in the group treated with CD18-DM1 or CD18-MMAE, the size of spheroids was decreased on day 8 (FIG. 37). In addition, as a result of confirming related markers by staining the spheroids with immunofluorescence on day 8 after drug treatment, it was shown that the fluorescence was strongly increased in the group not treated with ADC, whereas in the group treated with CD18-DM1 or CD18-MMAE, the number of THP-1 cells was significantly reduced, and the tumor proliferation factor ki-67 was also decreased (***p < 0.001) (FIG. 38).

## Claims

1. A pharmaceutical composition for removing tumor-associated macrophages (TAMs) in a tumor microenvironment, comprising as an active ingredient a conjugate in which a pro-apoptotic peptide or an anticancer drug is conjugated to melittin, a variant thereof, or analogues thereof.

2. The pharmaceutical composition of claim 1, wherein the tumor-associated macrophages are M2 tumor-associated macrophages expressing Integrin beta 2 (IGBT2).

3. The pharmaceutical composition of claim 1, wherein the melittin, the variant thereof or the analogues thereof specifically bind to an active conformation in which CD18 is extended.

4. The pharmaceutical composition of claim 1, wherein the melittin, the variant thereof or the analogues thereof bind to amino acids at positions 449, and 466 to 565 of ITGB2.

5. The pharmaceutical composition of claim 1, wherein the melittin includes an amino acid sequence of SEQ ID NO: 1.

6. The pharmaceutical composition of claim 1, wherein the variant of the melittin includes an amino acid sequence of SEQ ID NO: 2.

7. The pharmaceutical composition of claim 1, wherein the pharmaceutical composition is linked by a linker.

8. The pharmaceutical composition of claim 7, wherein the linker includes an amino acid sequence of SEQ ID NO: 3.

9. The pharmaceutical composition of claim 1, wherein the pharmaceutical composition includes a peptide including an amino acid sequence of SEQ ID NO: 5 or 6.

10. The pharmaceutical composition of claim 1, wherein the pro-apoptotic peptide is selected from the group consisting of KLA, alpha-defensin-1, BMAP-28, Brevenin-2R, Buforin IIb, cecropin A-Magainin 2 (CA-MA-2), Cecropin A, Cecropin B, chrysophsin-1, D-K6L9, Gomesin, Lactoferricin B, LLL27, LTX-315, Magainin 2, Magainin II-bombesin conjugate (MG2B), Pardaxin, and combinations thereof.

11. The pharmaceutical composition of claim 1, wherein the anticancer drug is selected from the group consisting of 7-ethyl-10-hydroxy-camptothecin (SN-38), daunorubicin, doxorubicin, epirubicin, idarubicin, pixantrone, sabarubicin, valrubicin, paclitaxel, docetaxel, mechloethamine, chlorambucil, phenylalanine, mustard, cyclophosphamide, ifosfamide, carmustine (BCNU), lomustine (CCNU), Streptozotocin, busulfan, thiotepa, cisplatin, carboplatin, dactinomycin (actinomycin D), plicamycin, mitomycin C, vincristine, vinblastine, teniposide, topotecan, iridotecan, uramustine, melphalan, bendamustine, dacarbazine, temozolomide, altretamine, duocarmycin, nedaplatin, oxaliplatin, satraplatin, triplatin tetranitrate, 5-fluorouracil, 6-mercaptopurine, capecitabine, cladribine, clofarabine, cystarbine, floxuridine, fludarabine, gemcitabine, hydroxyurea, methotrexate, pemetrexed, pentostatin, thioguanine, etoposide, mitoxantrone, izabepilone, vindesine, vinorelbine, estramustine, maytansine, mertansine (DM1), DM4, dolastatin, auristatin E, auristatin F, monomethyl auristatin E (MMAE), monomethyl auristatin F, and derivatives thereof.

12. A pharmaceutical composition for preventing or treating cancer, comprising as an active ingredient a conjugate in which a pro-apoptotic peptide or an anticancer drug is conjugated to melittin, a variant thereof, or analogues thereof.

13. The pharmaceutical composition for treating or preventing cancer of claim 12, wherein the cancer is any one selected from the group consisting of brain tumor, melanoma, myeloma, non-small cell lung cancer, oral cancer, liver cancer, stomach cancer, colon cancer, breast cancer, TNBC (Triple Negative Breast Cancer), lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph adenocarcinoma, bladder cancer, gallbladder cancer, endocrine adenocarcinoma, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem gliomas and pituitary adenomas.

14. The pharmaceutical composition for preventing or treating cancer of claim 12, wherein the cancer is anticancer drug-resistant cancer.

15. The pharmaceutical composition for preventing or treating cancer of claim 12, wherein the pharmaceutical composition is administered to a patient in whom the expression of ITGB2 is upregulated in M2 macrophages compared to M0 macrophages and M1 macrophages.

16. A method for treating cancer, comprising administering to a subject suffering from cancer a pharmaceutically effective amount of a conjugate in which a pro-apoptotic peptide or an anticancer drug is conjugated to melittin, a variant thereof, or analogues thereof.

17. A use of a conjugate in which a pro-apoptotic peptide or an anticancer drug is conjugated to melittin, a variant thereof, or analogues thereof for the preparation of a pharmaceutical composition for preventing or treating cancer.
